# NEW EUROPEAN PATENT SPECIFICATION

(11) **EP 1 633 296 B2**
(45) Date of publication and mention of the opposition decision: **08.02.2012**
(45) Mention of the grant of the patent: 13.08.2008
(21) Application number: 04710239.7
(22) Date of filing: 11.02.2004
(51) Int. Cl.: A61F 13/15, A61L 15/16

(54) **PERSONAL WEAR ARTICLE WITH WETNESS INDICATOR**
HYGIENEARTIKEL MIT FEUCHTIGKEITSANZEIGER
ARTICLE VESTIMENTAIRE PERSONNEL A INDICATEUR D'HUMIDITE

(30) Priority: 16.06.2003 US 462166
(43) Date of publication of application: 15.03.2006
(73) Proprietor: KIMBERLY-CLARK WORLDWIDE, INC., Neenah, WI 54956 (US)
(72) Inventor: OLSON, Christopher, P., Neenah, WI 54956 (US); WEBER, Shirlee, Ann, Neenah, WI 54956 (US); SAWYER, Lawrence, Howell, Neenah, WI 54956 (US); HUANG, Lei, Duluth, GA 30097 (US); PERKINS, Cheryl, A., Appleton, WI 54915 (US); KIM, Jaeho, Roswell, GA 30075 (US); FISH, Jeffrey, E., Dacula, GA 30019 (US); HUANG, Yanbin, Foster City, California 94404 (US)
(74) Representative: Mabey, Katherine Frances
(86) International application number: PCT/US2004/004066
(87) International publication number: WO 2005/004771

(56) References cited:
- EP-A- 0 203 715
- EP-A- 0 339 461
- EP-A- 0 538 535
- WO-A-03/057109
- WO-A1-98/43571
- WO-A1-98/43578
- WO-A1-03/047641
- DE-C- 19 745 878
- US-A- 4 231 370
- US-A- 4 449 979
- US-A- 4 761 258
- US-A- 4 834 733
- US-A- 4 994 037
- US-A- 5 601 542
- US-A- 5 681 298
- US-A- 5 702 376
- US-A- 6 159 591
- US-A1- 2002 052 587
- US-A1- 2003 100 872
- US-B1- 6 320 096
- US-B1- 6 465 710

## Description

### Background of the Invention

The present invention relates generally to articles intended for personal wear, and more particularly to such articles having a wetness indicator for alerting the wearer to the wearer's release of liquid body exudates into such articles.

Many articles intended for personal wear, e.g., such as diapers, training pants, feminine hygiene products, adult incontinence products, bandages, medical garments and the like are designed to be sufficiently absorbent to pull moisture from liquid body exudates including urine, menses, blood, etc. away from the wearer to reduce skin irritation caused by prolonged wetness exposure. In some instances, such as for toilet training children, there is a belief that children must be given a signal such as an uncomfortable and/or wet feeling against the skin to facilitate toilet training by making the child more aware that the act of urination has occurred. On the other hand, there is a counter-balancing concern about the possibility of skin irritations and rashes caused by prolonged wetness against the skin if the articles are less absorbent to allow the child to sense wetness. However, by making articles such as training pants so absorbent, it is difficult for the wearer to realize that an insult of the article has occurred.

To this end, some prior articles intended for personal wear during toilet training include means for alerting a child to urination without leaving a substantial amount of wetness against the skin. For example, one such article includes a temperature changing element that allows the wearer to feel a change in temperature against the skin upon urination, thereby alerting the wearer to the urination. However, where such articles contain a conventional superabsorbent material (SAM) for absorbing and retaining urine (e.g., to draw and keep the wetness away from the wearer's skin), the absorption of electrolyte-containing liquids such as urine may decrease the absorbency capabilities of the superabsorbent material.

For example, the absorption properties of a typical, commercially available SAM, i.e., sodium polyacrylate, in deionized water and in 0.9% by weight sodium chloride (NaCl) solution illustrate the effect that the electrolyte-containing liquid can have on some SAM. The sodium polyacrylate can absorb 146.2 grams (g) of deionized water per gram of SAM (g/g) at 0 Pa (0 psi), 103.8 g of deionized water per gram of polymer at 1931 Pa (0.28 psi), and 34.3 g of deionized water per gram of polymer of 4826 Pa (0.7 psi). In contrast, the same sodium polyacrylate is capable of absorbing only 43.5 g, 29.7 g, and 24.8 g of 0.9% aqueous NaCl at 0 Pa (0 psi), 1931 Pa (0.28 psi), and 4826 Pa (0.7 psi), respectively. The absorption capacity of SAMs for body fluids such as urine or menses is thus dramatically lower than for deionized water because such fluids contain electrolytes. Products are designed to absorb their target fluid by building in sufficient absorbent capacity to accommodate the expected insult amounts. These designs take into account the mitigation of superabsorbent capacity that results from the ionic strength of the insult fluid. However, where a temperature changing element in a product is also electrolytic (as is seen in the prior art), the negative effect on superabsorbent capacity may be even more severe. This loss of capacity can cause negative product performance, including premature leakage.

Also, for the wearer of such an article to receive a temperature sensation against the skin, a thermally conductive heat transfer must occur between the article and the wearer's skin, e.g., where there is direct contact between a heated or cooled layer of the article and the skin. The effects of convection and radiation are relatively insignificant in such an article. Thus, if there is any space between the heated or cooled layer and the wearer's skin, thermally conductive heat transfer cannot occur (e.g., there is more of a "thermos bottle" effect).

Another example of training pants intended to provide a sensory indication of urination includes an element that changes size after urination, e.g., expanding toward the wearer's crotch region. However, such elements are typically surrounded by highly absorbent structures (sometimes referred to as absorbent cores) which compete for and may draw urine away from the element, thereby prolonging or otherwise inhibiting the expansion thereof and diminishing its potential training effectiveness. Also, superabsorbent material (SAM) which is used to make the highly absorbent structures of such articles expands upon absorbing urine. Such expansion may mask or otherwise cushion the feeling of the expanded sensory element, thus making it difficult for the wearer to sense the intended signal.

Consequently, while there has been progress in the design of personal wear articles capable of alerting a wearer to a release of liquid body exudates, there continues to be a need for improvements in such articles.

### Summary of the Invention

The present invention provides an article for personal wear in accordance with claim 1.

The invention may provide an article for personal wear capable of alerting a wearer to the wearer's release of liquid body exudates. The article includes an absorbent structure adapted to absorb the liquid body exudates, the absorbent structure having an absorbent capacity gradient along at least a portion thereof. The article also includes a wetness indicator adapted to receive the liquid body exudates, the wetness indicator being responsive to the liquid body exudates to apply a tactile sensation against the wearer's skin. The wetness indicator is positioned relative to the portion of the absorbent structure having an absorbent capacity gradient such that the absorbent capacity of the absorbent structure increases as the absorbent structure extends generally from the position of the wetness indicator in a direction generally away from the position of the wetness indicator.

In embodiments, the wetness indicator is disposed in spaced relationship with the absorbent structure to define a gap therebetween.

In embodiments of the present invention, the absorbent body is capable of expansion upon the absorption of liquid body exudates thereby, the enclosure limiting the expansion of the absorbent body whereby the wetness indicator stiffens as the absorbent body absorbs the liquid body exudates. The wetness indicator may further include a temperature change agent responsive to the liquid body exudates to change the temperature of the liquid body exudates to thereby facilitate a temperature change sensation against the wearer's skin. In these embodiments, the temperature change agent is disposed at least one of on or within the liquid permeable enclosure such that liquid body exudates absorbed by the absorbent body of the wetness indicator are subjected to a temperature change by the temperature change agent at least one of prior to and upon absorption of the liquid body exudates by the absorbent body.

In another embodiment of the present invention, the article includes a liner adapted for contiguous relationship with the wearer's skin, an outer cover, and a wetness indicator disposed between the liner and the outer cover. The wetness indicator includes a liquid permeable enclosure and an absorbent body within the liquid permeable enclosure. The absorbent body is capable of expansion upon the absorption of the liquid body exudates, the enclosure limiting the expansion of the absorbent body whereby the wetness indicator stiffens as the absorbent body absorbs the liquid body exudates. The article also includes a temperature change agent responsive to the liquid body exudates to change the temperature thereof. The temperature change agent is disposed relative to the wetness indicator such that liquid body exudates absorbed by the absorbent body of the wetness indicator are subjected to a temperature change by the temperature change agent at least one of prior to and during absorption of the liquid body exudates by the absorbent body of the wetness indicator.

Other features of the invention will be in part apparent and in part pointed out hereinafter.

### Brief Description of the Drawings

Figure 1 is a side perspective of an article of the present invention shown in the form of a pair of training pants having a mechanical fastening system shown fastened on one side of the training pants and unfastened on the other side of the training pants;

Figure 2 is a bottom plan view of the training pants of Figure 1 in an unfastened, unfolded and laid flat condition, and showing the surface of the training pants that faces away from the wearer;

Figure 3 is a top plan view similar to Fig. 2 showing the surface of the training pants that faces the wearer when worn and with portions cut away to show underlying features;

Figure 4 is a schematic top plan view similar to Fig. 2 with a liner of the training pants omitted to reveal an absorbent structure and wetness indicator of the pants;

Figure 4A is a section taken in the plane of line 4A-4A of Fig. 4;

Figure 5 is a schematic top plan view similar to Fig. 4 but showing a second embodiment of an absorbent structure and wetness indicator of the pants;

Figure 5A is a section taken in the plane of line 5A-5A of Fig. 5;

Figure 6 is a schematic top plan view similar to Fig. 4 but showing a third embodiment of an absorbent structure and wetness indicator of the pants;

Figure 6A is a section taken in the plane of line 6A-6A of Fig. 6;

Figure 7 is an enlarged schematic front elevation of the wetness indicator of Fig. 4 with portions partially broken away to reveal internal construction;

Figure 8 is a section taken in the plane of line 8-8 of Fig. 7;

Figure 9 is a schematic top plan view similar to Fig. 4 but showing a fourth embodiment of an absorbent structure and wetness indicator along with a free-liquid holding member of the training pants;

Figure 10 is cross-section similar to Fig. 8 but showing a second embodiment of a wetness indicator; and

Figure 11 is a transverse cross-section of a third embodiment of a wetness indicator of the present invention.

Corresponding reference characters indicate corresponding parts throughout the several views of the drawings.

### Definitions

Within the context of this specification, each term or phrase below includes the following meaning or meanings:

"Attached to" refers to the joining, adhering, connecting, bonding, or the like, of two elements. Two elements will be considered to be attached to one another when they are attached directly to one another or indirectly to one another, such as when each is directly attached to intermediate elements.

"Free-liquid" refers to liquid that is not strongly held by capillary attraction, hydrogen bonding or other forces and is free to flow under the force of gravity and any pressure caused by the movements of the wearer or compression caused by the weight of the wearer.

"Hydrophilic" describes fibers or the surfaces of fibers which are wetted by aqueous liquids in contact with the fibers. The degree of wetting of the materials can, in turn, be described in terms of the contact angles and the surface tensions of the liquids and materials involved. Equipment and techniques suitable for measuring the wettability of particular fiber materials or blends of fiber materials can be provided by a Cahn SFA-222 Surface Force Analyzer System, or a substantially equivalent system. When measured with this system, fibers having contact angles less than 90 degrees are designated "wettable" or hydrophilic, and fibers having contact angles greater than 90 degrees are designated "nonwettable" or hydrophobic.

"Layer" when used in the singular can have the dual meaning of a single element or a plurality of elements.

"Liquid impermeable," when used in describing a layer or multi-layer laminate means that liquid body waste, such as urine, will not pass through the layer or laminate, under ordinary use conditions, in a direction generally perpendicular to the plane of the layer or laminate at the point of liquid contact.

"Liquid permeable" refers to any material that is not liquid impermeable.

"Meltblown" refers to fibers formed by extruding a molten thermoplastic material through a plurality of fine, usually circular, die capillaries as molten threads or filaments into converging high velocity heated gas (e.g., air) streams which attenuate the filaments of molten thermoplastic material to reduce their diameters. Thereafter, the meltblown fibers are carried by the high velocity gas stream and are deposited on a collecting surface to form a web of randomly dispersed meltblown fibers. Such a process is disclosed, for example, in U.S. Patent 3,849,241 to Butin et al. Meltblown fibers are microfibers which may be continuous or discontinuous, are generally smaller than about 0.6 denier, and are generally self bonding when deposited onto a collecting surface. Meltblown fibers used in the present invention are preferably substantially continuous in length.

"Non-woven" and "non-woven web" refer to materials and webs of material which are formed without the aid of a textile weaving or knitting process.

"Superabsorbent" refers to a water-swellable, water-insoluble organic or inorganic material capable, under the most favorable conditions, of absorbing at least about fifteen times its weight and, more desirably, at least about thirty times its weight in an aqueous solution containing 0.9 weight percent sodium chloride. The superabsorbent materials can be natural, synthetic and modified natural polymers and materials, or a combination of such.materials. In addition, the superabsorbent materials can be inorganic materials, such as silica gels, or organic compounds such as cross-linked polymers.

"Surge Layer" refers to a layer typically comprised of non-woven materials capable of absorbing a large surge of liquid and releasing it slowly into another layer or layers.

"Thermoplastic" describes a material which softens when exposed to heat and which substantially returns to a non-softened condition when cooled to room temperature.

### Detailed Description of the Preferred Embodiments

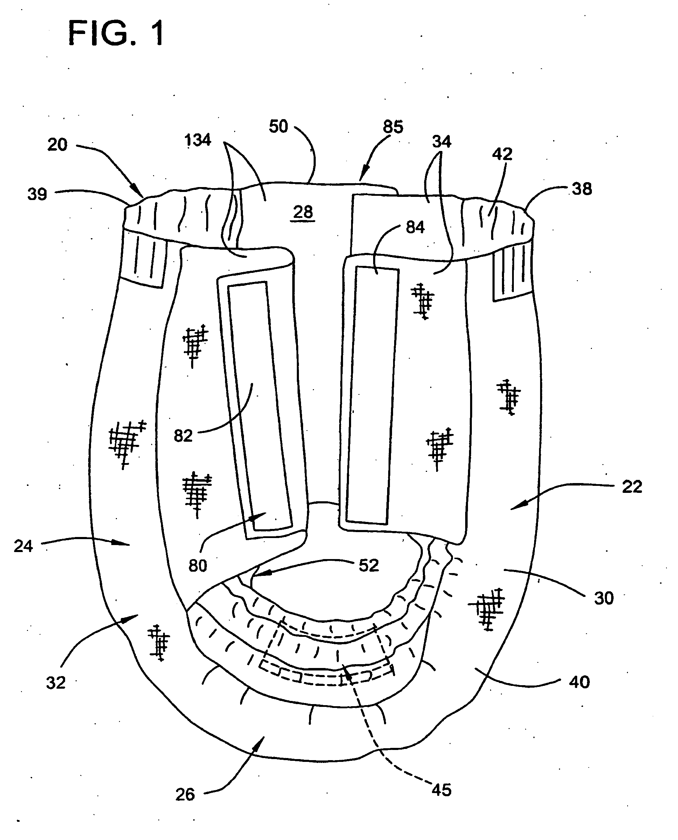

Referring now to the drawings and in particular to Fig. 1, an article of the present invention is shown therein in the form of children's toilet training pants and is indicated in its entirety by the reference numeral 20 and has a wetness indicator (shown in phantom and indicated generally at 45 in Fig. 1). The article may or may not be absorbent, which generally refers to absorbent articles that may be placed against or in proximity to the body of the wearer to absorb and/or retain various liquid wastes discharged from the body. The article also may or may not be disposable, which refers to articles that are intended to be discarded after a limited period of use instead of being laundered or otherwise restored for reuse. It is understood that the present invention, including the wetness indicator 45, is suitable for use with various other articles intended for personal wear such as clothing, diapers, feminine hygiene products, incontinence products, medical garments, surgical pads and bandages, other personal care or health care garments, and the like without departing from the scope of the present invention.

By way of illustration only, various materials and methods for constructing the training pants 20 are disclosed in PCT Patent Application WO 00/37009 published June 29, 2000 by A. Fletcher et al; U.S. Patent 4,940,464 issued July 10, 1990 to Van Gompel et al.; and U.S. Patent 5,766,389 issued June 16, 1998 to Brandon et al.

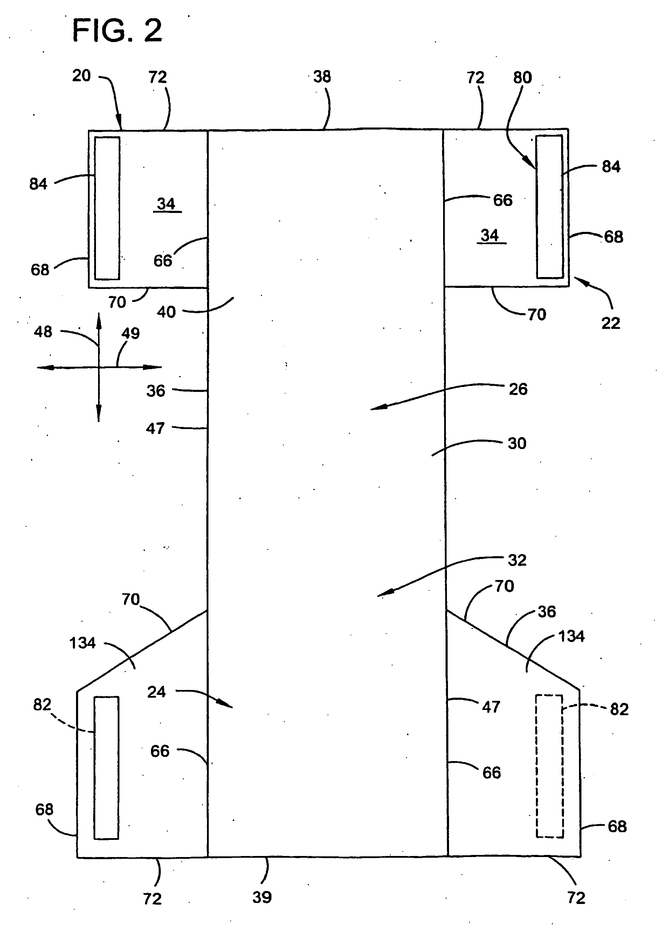

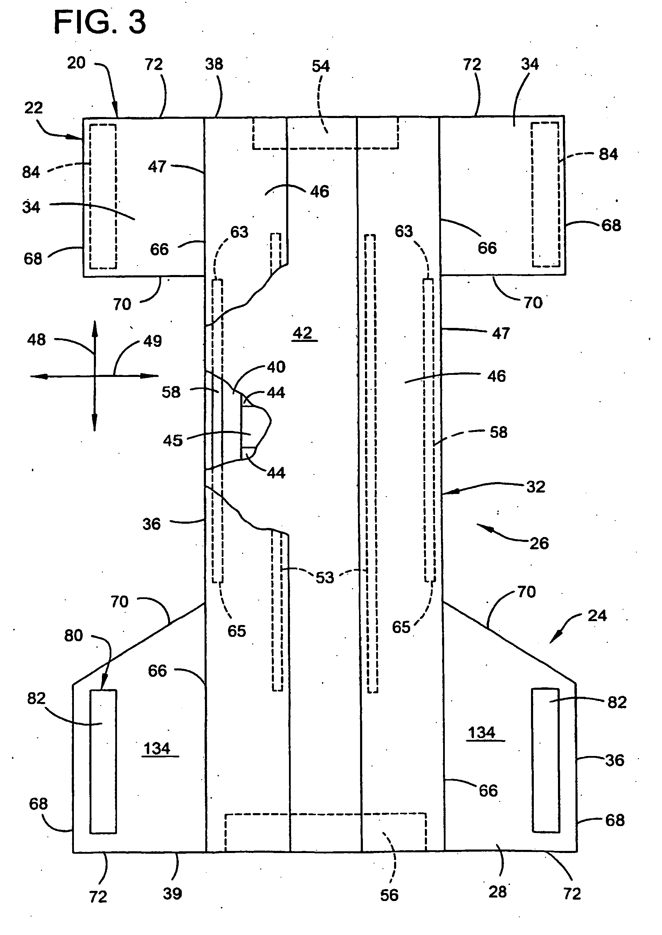

The pair of training pants 20 is illustrated in Fig. 1 in a partially fastened condition and comprises a front waist region 22, a back waist region 24, a crotch region 26 interconnecting the front and back waist regions, an inner surface 28 configured for contiguous relationship with the wearer, and an outer surface 30 opposite the inner surface. With additional reference to Figs. 2 and 3, the training pants 20, also has a pair of laterally opposite side edges 36 and a pair of longitudinally opposite waist edges, respectively designated front waist edge 38 and back waist edge 39. The front waist region 22 is contiguous with the front waist edge 38, and the back waist region 24 is contiguous with the back waist edge 39.

The illustrated pants 20 comprises a central absorbent assembly, generally indicated at 32, which when laid flat can be rectangular or any other desired shape, a pair of laterally opposite front side panels 34 extending outward therefrom at the front waist region 22 and a pair of laterally opposite back side panels 134 extending outward therefrom at the back waist region 24. The absorbent assembly 32 and side panels 34, 134 may comprise two or more separate elements, as shown in Fig. 1, or they may be integrally formed. The central absorbent assembly 32 of the illustrated embodiment comprises an outer cover 40 and a bodyside liner 42 (Figs. 1 and 3) connected to the outer cover in a superposed relation by suitable means such as adhesives, ultrasonic bonds, thermal bonds or other conventional techniques. An absorbent structure 44 (Fig. 3) is disposed between the outer cover and the bodyside liner along with the wetness indicator 45 (Fig. 3) as will be described later herein. A pair of containment flaps 46 (Fig. 3) is secured to the bodyside liner 42 for inhibiting the lateral flow of body exudates.

The central absorbent assembly 32 has opposite ends which form portions of the front and back waist edges 38 and 39, and opposite side edges 47 which form portions of the side edges 36 of the training pants 20 (Figs. 2 and 3). Integrally formed side panels 34, 134 and absorbent assembly 32 would comprise at least some common materials, such as the bodyside liner, flap composite, outer cover, other materials and/or combinations thereof, and could define a one-piece elastic, stretchable, or nonstretchable pants. For further reference, arrows 48 and 49 depict the orientation of the longitudinal axis and the transverse or lateral axis, respectively, of the training pants 20.

With the training pants 20 in the fastened position as partially illustrated in Fig. 1, the front and back side panels 34, 134 are connected together by a fastening system 80 to define a three-dimensional pants configuration having a waist opening 50 and a pair of leg openings 52. The front waist region 22 comprises the portion of the training pants 20 which, when worn, is positioned on the front of the wearer while the back waist region 24 comprises the portion of the training pants which is positioned on the back of the wearer. The crotch region 26 of the training pants 20 comprises the portion of the training pants 20 which is positioned between the legs of the wearer and covers the lower torso of the wearer. The front and back side panels 34 and 134 comprise the portions of the training pants 20 which, when worn, are positioned on the hips of the wearer. The waist edges 38 and 39 of the training pants 20 are configured to encircle the waist of the wearer and together define the waist opening 50 (Fig. 1). Portions of the side edges 36 in the crotch region 26 generally define the leg openings 52.

The central absorbent assembly 32 is configured to contain and/or absorb exudates discharged from the wearer. For example, the containment flaps 46 are configured to provide a barrier to the transverse flow of body exudates. A flap elastic member 53 (Fig. 3) can be operatively joined with each containment flap 46 in any suitable manner as is well known in the art. The elasticized containment flaps 46 define a partially unattached edge which assumes an upright configuration in at least the crotch region 26 of the training pants 20 to form a seal against the wearer's body. The containment flaps 46 can be located along the side edges 36 of the pants 20, and can extend longitudinally along the entire length of the absorbent assembly 32 or may only extend partially along the length of the absorbent assembly. Suitable constructions and arrangements for the containment flaps 46 are generally well known to those skilled in the art and are described in U.S. Patent 4,704,116 issued November 3, 1987 to Enloe.

To further enhance containment and/or absorption of body exudates, the training pants 20 also suitably includes a front waist elastic member 54 (Figure 3), a rear waist elastic member 56, and leg elastic members 58, as are known to those skilled in the art. The waist elastic members 54 and 56 can be operatively joined to the outer cover 40 and/or the bodyside liner 42 along the opposite waist edges 38 and 39, and can extend over part or all of the waist edges. The leg elastic members 58 can be operatively joined to the outer cover 40 and/or the bodyside liner 42 along the opposite side edges 36 and positioned in the crotch region 26 of the training pants 20. The leg elastic members 58 can be longitudinally aligned along each side edge 47 of the absorbent assembly 32. Each leg elastic member 58 has a front terminal point 63 and a back terminal point 65, which represent the longitudinal ends of the elastic gathering caused by the leg elastic members.

The flap elastic members 53, the waist elastic members 54 and 56, and the leg elastic members 58 can be formed of any suitable elastic material. As is well known to those skilled in the art, suitable elastic materials include sheets, strands or ribbons of natural rubber, synthetic rubber, or thermoplastic elastomeric polymers. The elastic materials can be stretched and adhered to a substrate, adhered to a gathered substrate, or adhered to a substrate and then elasticized or shrunk, for example with the application of heat, such that elastic retractive forces are imparted to the substrate. In one particular embodiment, for example, the leg elastic members 58 comprise a plurality of dry-spun coalesced multifilament spandex elastomeric threads sold under the trade name LYCRA^{®} and available from E. I. Du Pont de Nemours and Company, Wilmington, Delaware, U.S.A.

The outer cover 40 suitably comprises a material which is substantially liquid impermeable. The outer cover 40 can be a single layer of liquid impermeable material, but morse suitably comprises a multi-layered laminate structure in which at least one of the layers is liquid impermeable. For instance, the outer cover 40 can include a liquid permeable outer layer and a liquid impermeable inner layer that are suitably joined together by a laminate adhesive, ultrasonic bonds, thermal bonds, or the like. Suitable laminate adhesives, which can be applied continuously or intermittently as beads, a spray, parallel swirls, or the like, can be obtained from Findley Adhesives, Inc., of Wauwatosa, Wisconsin, U.S.A., or from National Starch and Chemical Company, Bridgewater, New Jersey U.S.A. The liquid permeable outer layer can be any suitable material and is desirably one that provides a generally cloth-like texture. One example of such a material is a 20 gsm (grams per square meter) spunbond polypropylene nonwoven web. The outer layer may also be made of those materials of which the liquid permeable bodyside liner 42 is made. While it is not a necessity for the outer layer to be liquid permeable, it is suitable that it provides a relatively cloth-like texture to the wearer.

The inner layer of the outer cover 40 can be both liquid and vapor impermeable, or it may be liquid impermeable and vapor permeable. The inner layer can be manufactured from a thin plastic film, although other flexible liquid impermeable materials may also be used. The inner layer, or the liquid impermeable outer cover 40 when a single layer, prevents waste material from wetting articles, such as bed sheets and clothing, as well as the wearer and caregiver. A suitable liquid impermeable film for use as a liquid impermeable inner layer, or a single layer liquid impermeable outer cover 40, is a 0.2 millimeter polyethylene film commercially available from Pliant Corporation of Schaumburg, Illinois, U.S.A.

If the outer cover 40 is a single layer of material, it can be embossed and/or matte finished to provide a more cloth-like appearance. As earlier mentioned, the liquid impermeable material can permit vapors to escape from the interior of the disposable absorbent article, while still preventing liquids from passing through the outer cover 40. A suitable "breathable" material is composed of a microporous polymer film or a nonwoven fabric that has been coated or otherwise treated to impart a desired level of liquid impermeability. A suitable microporous film is a PMP-1 film material commercially available from Mitsui Toatsu Chemicals, Inc., Tokyo, Japan, or an XKO-8044 polyolefin film commercially available from 3M Company, Minneapolis, Minnesota U.S.A.

The liquid permeable bodyside liner 42 is illustrated as overlying the outer cover 40, absorbent core 44 and wetness indicator 45, and may but need not have the same dimensions as the outer cover 40. The bodyside liner 42 is suitably compliant, soft feeling, and non-irritating to the child's skin. Further, the bodyside liner 42 can be less hydrophilic than the absorbent structure 44 to present a relatively dry surface to the wearer and permit liquid to readily penetrate through its thickness. Alternatively, the bodyside liner 42 can be more hydrophilic or can have essentially the same affinity for moisture as the absorbent structure 44 to present a relatively wet surface to the wearer to increase the sensation of being wet. This wet sensation can be useful as a training aid. The hydrophilic/hydrophobic properties can be varied across the length, width and/or depth of the bodyside liner 42 and absorbent structure 44 to achieve the desired wetness sensation or leakage performances.

The bodyside liner 42 can be manufactured from a wide selection of web materials, such as synthetic fibers (for example, polyester or polypropylene fibers), natural fibers (for example, wood or cotton fibers), a combination of natural and synthetic fibers, porous foams, reticulated foams, apertured plastic films, or the like. Various woven and nonwoven fabrics can be used for the bodyside liner 42. For example, the bodyside liner can be composed of a meltblown or spunbonded web of polyolefin fibers. The bodyside liner can also be a bonded-carded web composed of natural and/or synthetic fibers. The bodyside liner can be composed of a substantially hydrophobic material, and the hydrophobic material can, optionally, be treated with a surfactant or otherwise processed to impart a desired level of wettability and hydrophilicity. For example, the material can be surface treated with about 0.45 weight percent of a surfactant mixture comprising Ahcovel N-62 from Hodgson Textile Chemicals of Mount Holly, North Carolina, U.S.A. and Glucopan 220UP from Henkel Corporation of Ambler, Pennsylvania in an active ratio of 3:1. The surfactant can be applied by any conventional means, such as spraying, printing, brush coating or the like. The surfactant can be applied to the entire bodyside liner 42 or can be selectively applied to particular sections of the bodyside liner, such as the medial section along the longitudinal center line.

A suitable liquid permeable bodyside liner 42 is a nonwoven bicomponent web having a basis weight of about 27 gsm. The nonwoven bicomponent can be a spunbond bicomponent web, or a bonded carded bicomponent web. Suitable bicomponent fibers include a polyethylene/polypropylene bicomponent fiber available from CHISSO Corporation, Osaka, Japan. In this particular bicomponent fiber, the polypropylene forms the core and the polyethylene forms the sheath of the fiber. Other fiber orientations are possible, such as multi-lobe, side-by-side, end-to-end, or the like.

As noted previously, the illustrated training pants 20 have front and back side panels 34 and 134 disposed on each side of the absorbent assembly 32. The side panels 34, 134 can be permanently bonded along seams 66 to the central absorbent assembly 32 in the respective front and back waist regions 22 and 24. More particularly, as seen best in Figs. 2 and 3, the front side panels 34 can be permanently bonded to and extend transversely outward beyond the side edges 47 of the absorbent assembly 32 in the front waist region 22, and the back side panels 134 can be permanently bonded to and extend transversely outward beyond the side edges of the absorbent assembly in the back waist region 24. The side panels 34 and 134 may be bonded to the absorbent assembly 32 using attachment means known to those skilled in the art such as adhesive, thermal or ultrasonic bonding. Alternatively, the side panels 34 and 134 can be formed as an integral portion of a component of the absorbent assembly 32. For example, the side panels can comprise a generally wider portion of the outer cover 40, the bodyside liner 42, and/or another component of the absorbent assembly 32. The front and back side panels 34 and 134 can be permanently bonded together or be releasably connected with one another such as by the fastening system 80 of the illustrated embodiment.

The front and back side panels 34, 134 each have an outer edge 68 spaced laterally from the seam 66, a leg end edge 70 disposed toward the longitudinal center of the training pants 20, and a waist end edge 72 disposed toward a longitudinal end of the training pants. The leg end edge 70 and waist end edge 72 extend from the side edges 47 of the absorbent assembly 32 to the outer edges 68. The leg end edges 70 of the side panels 34 and 134 form part of the side edges 36 of the training pants 20. The leg end edges 70 of the illustrated embodiment are suitably curved and/or angled relative to the transverse axis 49 to provide a better fit around the wearer's legs. However, it is understood that only one of the leg end edges 70 may be curved or angled, such as the leg end edge of the back waist region 24, or neither of the leg end edges may be curved or angled, without departing from the scope of this invention. The waist end edges 72 are suitably parallel to the transverse axis 49. The waist end edges 72 of the front side panels 34 form part of the front waist edge 38 of the training pants 20, and the waist end edges 72 of the back side panels 134 form part of the back waist edge 39 of the pants.

The side panels 34, 134 suitably, although not necessarily, comprise an elastic material capable of stretching in a direction generally parallel to the transverse axis 49 of the training pants 20. suitable elastic materials, as well as one process of incorporating elastic side panels into training pants, are described in the following U.S. Patents: 4,940,464 issued July 10, 1990 to Van Gompel et al.; 5,224,405 issued July 6, 1993 to Pohjola; 5,104,116 issued April 14, 1992 to Pohjola; and 5,046,272 issued September 10, 1991 to Vogt et al. In particular embodiments, the elastic material may comprise a stretch-thermal laminate (STL), a neck-bonded laminate (NBL), a reversibly necked laminate, or a stretch-bonded laminate (SBL) material. Methods of making such materials are well known to those skilled in the art and described in U.S. Patent 4,663,220 issued May 5, 1987 to Wisneski et al.; U.S. Patent 5,226,992 issued July 13, 1993 to Morman; European Patent Application No. EP 0 217 032 published on April 8, 1987 in the name of Taylor et al.; and PCT application WO 01/88245 in the name of Welch et al. Alternatively, the side panel material may comprise other woven or nonwoven materials, such as those described above as being suitable for the outer cover 40 or bodyside liner 42; mechanically pre-strained composites; or stretchable but inelastic materials.

The fastening system 80 comprises laterally opposite first fastening components 82 adapted for refastenable engagement to corresponding second fastening components 84. In one embodiment, a front or outer surface of each of the fastening components 82, 84 comprises a plurality of engaging elements. The engaging elements of the first fastening components 82 are adapted to repeatedly engage and disengage corresponding engaging elements of the second fastening components 84 to releasably secure the pants 20 in its three-dimensional configuration.

The fastening components 82, 84 can comprise any refastenable fasteners suitable for absorbent articles, such as adhesive fasteners, cohesive fasteners, mechanical fasteners, or the like. In particular embodiments the fastening components comprise mechanical fastening elements for improved performance. Suitable mechanical fastening elements can be provided by interlocking geometric shaped materials, such as hooks, loops, bulbs, mushrooms, arrowheads, balls on stems, male and female mating components, buckles, snaps, or the like.

In the illustrated embodiment, the first fastening components 82 comprise loop fasteners and the second fastening components 84 comprise complementary hook fasteners. Alternatively, the first fastening components 82 may comprise hook fasteners and the second fastening components 84 may comprise complementary loop fasteners. In another embodiment, the fastening components 82, 84 can comprise interlocking similar surface fasteners, or adhesive and cohesive fastening elements such as an adhesive fastener and an adhesive-receptive landing zone or material; or the like. Although the training pants 20 illustrated in Fig. 1 show the back side panels 134 overlapping the front side panels 34 upon connection thereto, which is convenient, the training pants 20 can also be configured so that the front side panels overlap the back side panels when connected. One skilled in the art will recognize that the shape, density and polymer composition of the hooks and loops may be selected to obtain the desired level of engagement between the fastening components 82, 84. A more aggressive hook material may comprise a material with a greater average hook height and/or a greater percentage of directionally-aligned hooks. When engaged, the fastening components 82, 84 of the illustrated embodiment define refastenable engagement seams 85 (Fig. 1).

The absorbent structure 44 can be any structure which is generally compressible, conformable, non-irritating to the wearer's skin and capable of absorbing and retaining liquid body exudates, and may be manufactured in a wide variety of sizes and shapes, and from a wide variety of absorbent materials commonly used in the art. For example, the absorbent structure 44 suitably comprises a matrix of absorbent fibers, and more particularly hydrophilic fibers, such as a web of cellulosic fluff. In a particularly suitable embodiment, the absorbent structure 44 comprises a matrix of cellulosic fluff, such as wood pulp fluff, and superabsorbent particles. The wood pulp fluff can be exchanged with synthetic, polymeric, meltblown fibers or short cut homofil bicomponent synthetic fibers and natural fibers. The superabsorbent particles can be substantially homogeneously mixed with the hydrophilic fibers or can be nonuniformly mixed. The fluff and superabsorbent particles can also be selectively placed into desired zones of the absorbent structure 44 to better contain and absorb body exudates. The concentration of the superabsorbent particles can also vary through the thickness of the absorbent core 44. Alternatively, the absorbent structure 44 can comprise a laminate of fibrous webs and superabsorbent material or other suitable means of maintaining a superabsorbent material in a localized area.

Suitable superabsorbent materials can be selected from natural, synthetic, and modified natural polymers and materials. The superabsorbent materials can be inorganic materials, such as silica gels, or organic Compounds, such as crosslinked polymers, for example, sodium neutralized polyacrylic acid. Suitable superabsorbent materials are available from various commercial vendors, such as Dow Chemical Company located in Midland, Michigan, U.S.A. and Stockhausen GmbH & Co. KG, D-47805 Krefeld, Federal Republic of Germany.

In one embodiment, the absorbent structure 44 comprises a blend of wood pulp fluff and superabsorbent material. One preferred type of pulp fluff is identified with the trade designation CR1654, commercially available from U.S. Alliance, Childersburg, Alabama, U.S.A., and is a bleached, highly absorbent sulfate wood pulp containing primarily soft wood fibers and about 16 percent hardwood fibers. As a general rule, the superabsorbent material is present in the absorbent structure 44 in an amount of from 0 to about 90 weight percent based on total weight of the absorbent assembly. The absorbent structure 44 suitably has a density within the range of about 0.10 to about 0.35 grams per cubic centimeter. The absorbent structure 44 may or may not be wrapped or encompassed by a suitable tissue wrap that may help maintain the integrity and/or shape of the absorbent assembly.

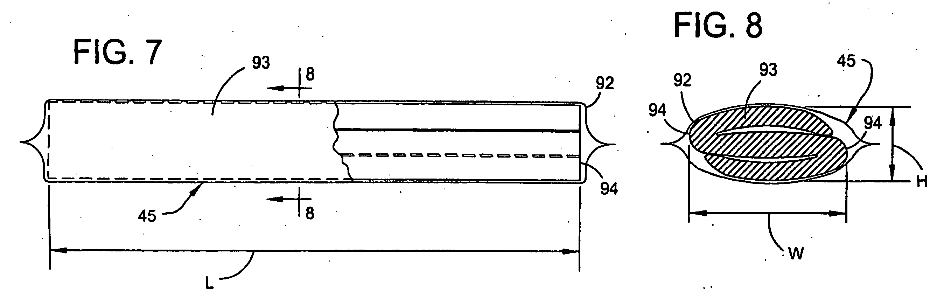

With particular reference to Figs. 7 and 8, the wetness indicator 45 suitably comprises a liquid permeable enclosure 92 having a liquid absorbent body 93 enclosed within the interior of the enclosure. Suitable liquid permeable enclosures 92 and absorbent bodies 93 for use in forming the wetness indicator 45 are described in co-assigned U.S. Patent Serial No. 10/038,863, filed December 31, 2001 by Olson, et al. and entitled WETNESS INDICATOR FOR ALERTING A WEARER TO URINATION. In one embodiment, the absorbent body 93 of the wetness indicator 45 comprises a sheet material folded over upon itself at least once to form at least two layers. The absorbent body 93 may have at least one longitudinal fold line 94, thereby forming at least two layers of absorbent sheet material. In the illustrated embodiment, the absorbent body 93 is formed from sheet material folded over upon itself twice along longitudinal fold lines 94 to form a three layer folded structure. Such a "fan-folded" structure enhances the strength of the absorbent body 93 since each fold layer will act to support adjacent fold layers, further magnifying the expansive pressure within the enclosure 92. As such, the overall strength and stiffness of the absorbent body 93 increases in the direction of the fold lines 94. By varying the number of fold lines 94 and the width of the layers, absorbent bodies 93 of different sizes and/or shapes can be made.

The absorbent body 93 is suitably formed from a high-absorbency-under-load/high-gel-strength materials. For example, the absorbent body may be formed from an Ultra Thin Absorbent (UTA) material comprising about fifty percent superabsorbent material and about fifty percent wood pulp. In a particular embodiment, such a UTA material has a basis weight of about 225 grams per square meter (gsm) at a thickness of about 0.65 millimeters. One such UTA material can be produced using an online process such as that described in U.S: Application number 09/939,061, entitled "Thin, High Capacity Absorbent Structure and Method for Producing Same," filed August 24, 2001, by Sawyer, et al. In addition, U.S. Patent No. 5,147,343 issued to Kellenberger and 5,601,542 issued to Melius et al. describe other processes for producing suitable UTA material. An alternative absorbent body material is available from EAM Corporation of Jessup, Georgia, under the trademark NovaThin^{®} material. In the illustrated embodiment, the wetness indicator 45 has a dry length of about 89 mm, a dry width of about 51 mm and dry thickness (e.g., height H) of about 4 mm, and has an absorbent body 93 constructed of a 550 gsm basis weight UTA material folded into three layers.

In one embodiment, the liquid permeable enclosure 92 is suitably formed from a 20 gsm spunbond nonwoven material available from Kimberly-Clark Corporation, Neenah, Wisconsin. More particularly, a pair of opposed sheets of such material are ultrasonically bonded together along an edge margin about the periphery of the wetness indicator 45, with the absorbent body 93 disposed between the sheets, so as to seal the absorbent body within the enclosure. The enclosure material may also be surface treated with about 0.45 weight percent of a surfactant mixture comprising Ahcovel N-62 from Hodgson Textile Chemicals of Mount Holly, North Carolina, U.S.A. The surfactant may be applied to the entire surface of the spunbond nonwoven material, or it may be selectively applied to particular surface portions, such as to a surface portion facing the body. It is contemplated that the enclosure 92 could also include liquid impermeable portions, such as those portions positioned away from the wearer without departing from the scope of the present invention. Also, it is understood that materials other than a spunbond nonwoven material may be used to form the enclosure 92 without departing from the scope of the present invention, as long as at least a portion of the enclosure is sufficiently liquid permeable to permit liquid body exudates to permeate therethrough into the interior of the enclosure for contact with and subsequent absorption by the absorbent body 93.

It is contemplated that the absorbent body 93 may alternatively be constructed of a single web or other structure that remains unfolded, or it may be constructed of multiple, separate layers of material overlaid upon each other (e.g., as opposed to a single sheet folded over upon itself) without departing from the scope of this invention.

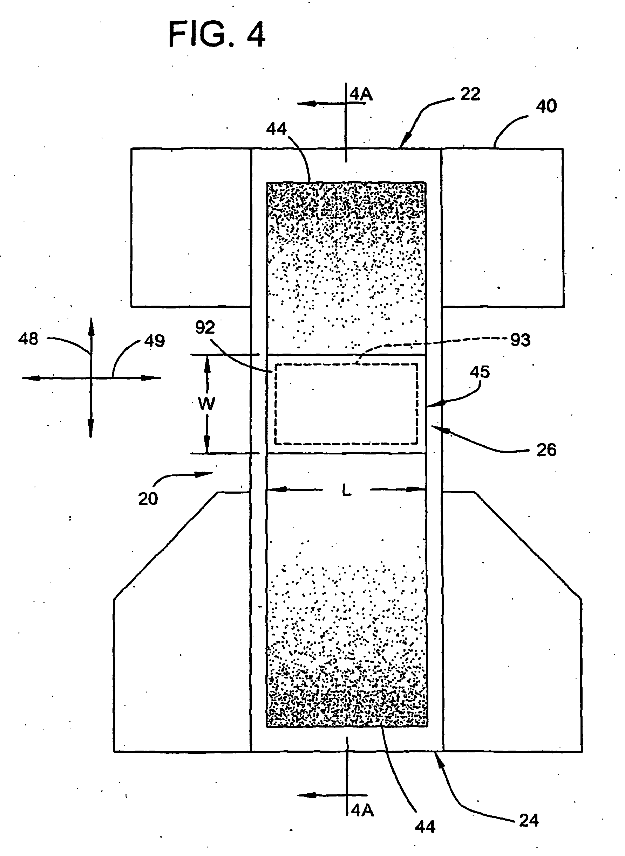

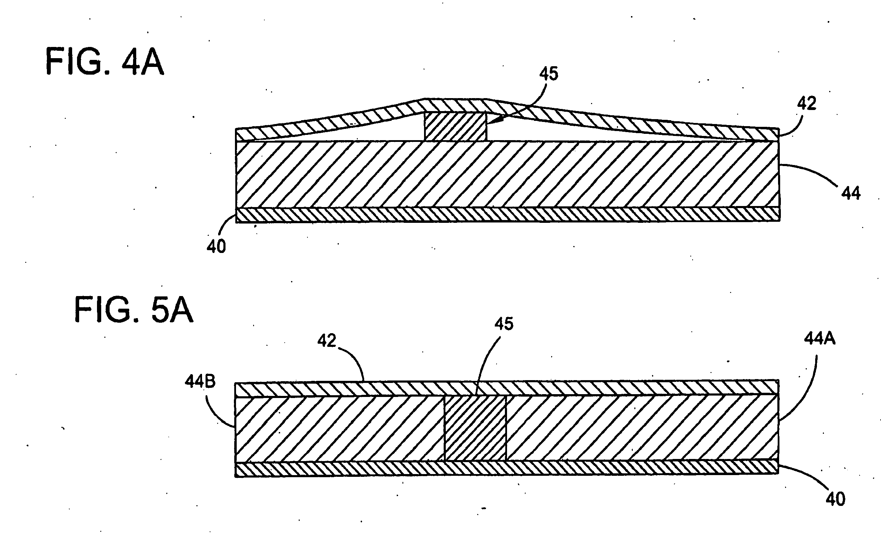

With particular reference now to Figs. 4 and 4a, the wetness indicator 45 is suitably disposed between the bodyside liner 42 and the outer cover 40 so that the wetness indicator is substantially imperceptible to the wearer prior to the first insult of the pants 20 by liquid body exudates, e.g., in the case of training pants, urine. The wetness indicator 45 is longitudinally positioned in the crotch region 26 of the training pants 20 between the leg openings 52 (Fig. 1) thereof. However, it is contemplated that the longitudinal position of the wetness indicator 45 within the crotch region 26 may be dependant on whether the pair of pants 20 is to be worn by a boy or a girl. For example, placement of the wetness indicator 45 in a more forward location within the crotch region 26 may be appropriate for boys, while placement in a more central location within the crotch region may be more appropriate for girls. It is also understood that the wetness indicator 45 may be positioned other than in the crotch region 26 without departing from the scope of the present invention, as long as the wetness indicator is suitably positioned so as to become wet and perceptible by a wearer upon insult of the pants by liquid body exudates.

Also, the wetness indicator 45 may be disposed other than between the liner and the outer cover, e.g., it may be disposed on the liner for direct contact with the wearer's skin and remain within the scope of this invention.
In such an embodiment, the wetness indicator may be attached to the liner, such as by being adhesively attached or bonded thereto, or it may be releasably secured to the liner to permit the caregiver to position the wetness indicator on the liner in a desired longitudinal position depending on whether the pants are to be worn by a boy or a girl.

While a single wetness indicator 45 is shown in the illustrated embodiment, it is contemplated that additional wetness indicators may be used to further enhance the signal to the wearer. For example, additional wetness indicators 45 may be necessary for larger or older children with larger legs for whom the resistive force provided by a single wetness indicator is generally insufficient to alert the wearer to his or her urination. A pair of wetness indicators 45 may also be used in a configuration wherein one wetness indicator is positioned longitudinally where it is more likely to become wet upon urination by boys and the other wetness indicator is positioned longitudinally where it is more likely to become wet upon urination by girls, thereby accounting for differences between the target wetting areas of boys and girls.

The wetness indicator 45 of the illustrated embodiment suitably extends transversely across the majority of the crotch region 26 of the pants 20, having a length L substantially equal to the width of the absorbent structure 44. Also, the wetness indicator 45 shown in Fig. 4 has a width W that is less than the length L (e.g., transverse extension) of the wetness indicator. As an example, the length L of the wetness indicator 45 is suitably in the range of about 50 mm to about 130 mm, and more suitably in the range of about 70 mm to about 100 mm. The width W is suitably in the range of about 20 mm to about 100 mm, and more suitably about 30 mm to about 60 mm. As a further example, a percent ratio of the width W divided by the length L (e.g., (W ÷ L) X 100) of the wetness indicator 45 is suitably in the range of from about 40 to about 80 percent, and is more suitably about 60 percent. As an example of relative dimensions, the crotch region 26 of the pants 20 of Fig. 4 has a width of approximately 109 mm, the absorbent structure 44 has a width at the crotch region of about 89 mm, and the wetness indicator 45 has a length L of about 89 mm and a width W of about 51 mm. However, it is understood that the wetness indicator 45 may be shorter or longer than the width of the absorbent structure 44. Also, the longitudinal extent of the wetness indicator 45 may be greater than or equal to the transverse extent thereof, or it may be less than that shown in Fig. 4, without departing from the scope of this invention.

the thickness, or height H (Fig. 8) of the wetness indicator 45 when dry is suitably in the range of about 2 mm to about 15 mm, and more suitably in the range of about 3 mm to about 9 mm. Upon absorption, the thickness or height H of the wetness indicator suitably expands to at least about 2 times its height when dry, and more suitably it expands to at least about 6 times the height when dry.

Still referring to Figs. 4 and 4A, the absorbent structure 44 extends longitudinally from generally within the front region 22 of the pants 20 through the crotch region 26 to generally within the back region 24 of the pants. The wetness indicator 45 suitably overlays the absorbent structure 44 within the crotch region 26. However, it is contemplated that the wetness indicator 45 may alternatively underlie the absorbent structure 44 or it may be enclosed within the absorbent structure, e.g., with a portion of the absorbent structure overlaying the wetness indicator and a portion of the absorbent structure underlying the wetness indicator, as long as the absorbent structure and wetness indicator are in generally overlaid relationship with each other. The wetness indicator 45 may be attached to the absorbent structure 44, and/or to the bodyside liner 42 (or to the outer cover where the wetness indicator underlies the absorbent structure), such as by ultrasonic bonding, adhesives, thermal bonds, or other suitable attachment techniques.

The absorbent structure 44 suitably has a non-uniform absorbent capacity along at least a portion of its length as indicated for illustrative purposes only by the varied dot density on the absorbent structure shown in Fig. 4. More particularly, the absorbent capacity of the absorbent structure 44 suitably increases longitudinally as the absorbent structure extends in a direction generally away from the position of the wetness indicator. As used herein, the position of the wetness indicator refers to the longitudinal and lateral center of the wetness indicator within the pants 20. Thus, the absorbent capacity of the absorbent structure 44 may increase from the center of the wetness indicator 45 outward therefrom (e.g., longitudinally or longitudinally and laterally), and/or it may increase as the absorbent structure extends beyond the edges of the wetness indicator, toward the front and back regions 22, 24 of the pants 20. It is contemplated that the absorbent capacity of the absorbent structure 44 may increase as the absorbent structure extends in only one direction, e.g., toward the front region 22 of the pants 20 where the target wetting area for boys is located. The absorbent capacity gradient defined by the rion-uniform absorbent capacity may be stepped or it may be gradual or otherwise non-stepped.

As used herein, the term absorbent capacity broadly refers to the ability of the absorbent structure 44 to take-in and/or retain liquid body exudates therein. The lower absorbent capacity of the absorbent structure in the region at and adjacent to the position of the wetness indicator 45 more readily facilitates the flow of liquid body exudates, such as via gravity, toward the wetness indicator. The size of the region of lower absorbent capacity, i.e., the longitudinal position at which the absorbent capacity increases (if the gradient is a stepped gradient) or the rate at which the absorbent capacity increases (where the gradient is gradual or otherwise non-stepped) depends generally on the amount of liquid body exudates required to flow to the wetness indicator 45 to activate the wetness indicator and the amount of liquid body exudates typically exuded by the wearer which must be taken into the pants 20 (e.g., by both the absorbent structure 44 and the wetness indicator), particularly during the first insult.

As an example, in one embodiment an absorbent capacity gradient may be formed in the absorbent structure 44 by increasing the density of the absorbent structure as the absorbent structure extends in a direction generally away from (e.g., in the illustrated embodiment, longitudinally away from) the position of the wetness indicator 45. That is, the density of the absorbent structure 44 at and/or generally longitudinally adjacent to the wetness indicator 45 is substantially less than the density of the absorbent structure as the absorbent structure extends longitudinally further away from the position of the wetness indicator. The decreased density nearer the wetness indicator 45 reduces the absorbent capacity of the absorbent structure 44 near the wetness incidator, thereby facilitating the flow of liquid to the wetness indicator. As an example, the density of the absorbent structure 44 in the crotch region 26 at and generally adjacent to the wetness indicator 45 is suitably in the range of about 0.1 to about 0.5 grams per cubic centimeter, and more suitably in the range of about 0.2 to about 0.4 grams per cubic centimeter. The density of the absorbent structure 44 increases as the absorbent structure extends longitudinally away from the position of the wetness indicator 45 to a density in the range of about 0.2 to about 0.6 grams per cubic centimeter, and more suitably in the range of about 0.25 to about 0.5 grams per cubic centimeter.

Alternatively, or additionally, the absorbent capacity gradient may be created by providing a relatively lower concentration of superabsorbent material at and/or adjacent to the wetness indicator 45 and increasing the concentration of superabsorbent material as the absorbent structure extends longitudinally away from the position of the wetness indicator. As an example, the concentration of superabsorbent material in the area of the wetness indicator 45 is suitably in the range of about zero to about 70 percent by weight of the absorbent structure 44, and more suitably in the range of about zero to about 50 weight percent. The concentration of superabsorbent material in the absorbent structure 44 suitably increases as the absorbent structure extends longitudinally away from the position of the wetness indicator 45 to a concentration in the range of about 30 to about 95 weight percent, and more suitably in the range of about 40 to about 90 weight percent.

Suitable techniques for forming absorbent structures having absorbent capacity gradients are known to those skilled in the art and will not be described herein. As an example, co-assigned U.S. Patent No. 4,761,258 entitled CONTROLLED FORMATION OF LIGHT AND HEAVY FLUFF ZONES by K. Enloe which issued August 2, 1988; U.S. Patent No. 6,330,735, entitled APPARATUS AND PROCESS FOR FORMING A LAID FIBROUS WEB WITH ENHANCED BASIS WEIGHT CAPABILITY by J. T. Hahn et al. which issued December 18, 2001; and U.S. Patent Application Serial No. 10/207,929 entitled APPARATUS AND FORM FOR MAKING AN AIR FORMED FIBROUS WEB by Venturino et al., filed July 30, 2002 (attorney docket No. 16,900), disclose examples of suitable techniques.

In use, the wetness indicator 45 is generally soft, pliable and cloth-like when dry and has a stiffness generally similar to that of other portions of the pants 20, and more particularly the absorbent structure 44, making the presence of the wetness indicator generally imperceptible to the wearer prior to urination. The pliable wetness indicator 45 allows the thighs of the wearer to move freely and to readily compress the wetness indicator 45 during normal movements. Upon the first insult of liquid body exudates, such as urine, the liquid permeates through the bodyside liner 42. The absorbent capacity gradient of the absorbent structure 44 facilitates the flow of at least some of the liquid, e.g., by gravity, toward the wetness indicator 45 whereby the liquid is drawn through the liquid permeable enclosure 92 of the wetness indicator 45, such as by capillary, osmotic and absorptive forces, for absorption by the absorbent body 93 within the enclosure.

As the wetness indicator 45 absorbs liquid, the absorbent body 93 within the enclosure 92 begins to swell. This swelling of the absorbent body 93 applies a hydraulic pressure against the enclosure 92. The unrestrained saturated volume of the absorbent body 93 is suitably greater than the volume of the liquid permeable enclosure 92 so that the enclosure limits expansion of the absorbent body therein, thereby causing the wetness indicator 45 to stiffen. If the liquid permeable enclosure 92 were too expandable, absorption of the liquid by the absorbent body 93 would simply expand the wetness indicator 45 without causing it to stiffen or otherwise lose some of its pliability. For example, the stiffness of the wetness indicator upon the absorption of liquid suitably increases to at least about 5 times the stiffness of the wetness indicator when dry, and more suitably it increases in the range of about 5 to more than 8 times the stiffness of the wetness indicator when dry. For comparison purposes between different wetness indicators, the stiffness is suitably measured upon the absorption by the wetness indicator of approximately 10 grams weight of urine or urine simulant per 1 gram weight of the wetness indicator.

The increased stiffness of the wetness indicator 45 provides a resistance to bending, folding, creasing, flexing, etc. of the pants 20, particularly in the crotch region 26, that may be readily perceived by the wearer. For example, the increased stiffness may resist movement by the wearer to bring the thighs of the wearer closer together. Such resistance against the inner thighs is need not be large, but is suitably sufficient to at least gently resist leg movement of the inside of the legs of the wearer such that the wearer perceives the resistance, which the wearer eventually learns to associate with urination.

Also, the liquid body exudates suitably remain within the enclosure 92 even under external pressure, such as applied by the legs of the wearer during normal movement. That is, the wetness indicator 45 suitably inhibits the flow back of liquid out of the absorbent body 93 and the enclosure 92 upon the application of compressive pressure to the wetness indicator, such as encountered during sitting, walking, twisting, etc. by the wearer, to thereby prolong the duration over which the increased stiffness of the wetness indicator may be perceived by the wearer.

The permeability and wettability of the bodyside liner 42 is suitably balanced so that substantially all of the first liquid insult of the article does not immediately pass through the liner into the absorbent core 44. When the wearer is sitting or standing, this delay in penetration of the bodyside liner results in the force of gravity drawing the liquid down into the crotch region 26 of the pants 20 toward the wetness indicator 45 rather than being distributed to other portions of the absorbent structure 44. This channeling mechanism is particularly useful in articles such as training pants 20 worn by boys because urine usually exits the urethra further from the crotch region 26 (where the wetness indicator 45 is positioned) than for girls. Thus, a greater amount of urine from the first insult reaches the absorbent body 93 of the wetness indicator 45 to generate stiffness in the wetness indicator so that the child gets tactile feedback after the first insult. This helps the child more closely associate the cause (urination) with the effect (uncomfortable pants 20).

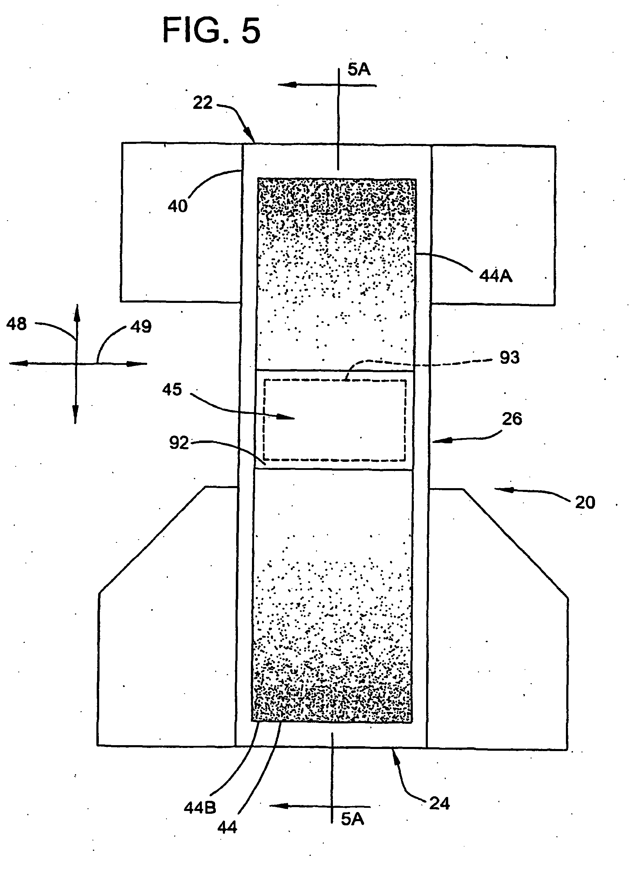

Figures 5 and 5a illustrate another embodiment which is similar to that of Fig. 4 but wherein the absorbent structure 44 is suitably of two-piece construction, with absorbent structure members 44A and 44B spaced longitudinally from each other and the wetness indicator 45 positioned longitudinally between the absorbent structure members in edge-facing-edge and generally abutting relationship therewith. One of the absorbent structure members 44A extends forward from the wetness indicator 45 toward the front region 22 of the pants 20 and the other absorbent structure member 44B extends rearward from the wetness indicator toward the back region 24 of the pants. The front absorbent structure member 44A suitably has a non-uniform absorbent capacity, and more particularly, it has an absorbent capacity gradient that increases from the edge abutting the wetness indicator in a direction away from (e.g., in the illustrated embodiment, longitudinally away from) the position of the wetness indicator, and more particularly from the edge of the wetness indicator.

The back absorbent structure member 44B also suitably has a non-uniform absorbent capacity, and more particularly, it has an absorbent capacity gradient that increases from the edge abutting the wetness indicator 45 in a direction away from (e.g., in the illustrated embodiment, longitudinally away from) the position of the wetness indicator, and more particularly from the edge of the wetness indicator. It is contemplated, however, that only one of the absorbent structure members 44A, 44B may have a non-uniform absorbent capacity without departing from the scope of this invention. For example, the front absorbent structure member 44A may have a non-uniform absorbent capacity corresponding to the target wetting area, such as by a boy, while the back absorbent structure member 44B may have a generally uniform absorbent capacity or otherwise an absorbent capacity that does not increase as the member extends away from the position of the wetness indicator.

The absorbent capacity gradient of each of the absorbent structure members 44A, 44B is suitably formed in any of the manners described previously for the absorbent structure 44 of Fig. 4. In the illustrated embodiment of Fig. 5A, the thickness of the wetness indicator 45 is substantially the same as that of the absorbent structure members 44A, 44B. However, it is understood that the thickness of the wetness indicator 45 may be different from (e.g., greater than or less than) that of the absorbent structure members 44A, 44B without departing from the scope of this invention. It is also understood that while the length (e.g., transverse extension) of the wetness indicator 45 is shown in Fig. 5 as being substantially the same as the width of the absorbent structure members 44A, 44B, the length may be shorter or longer than the width of the absorbent structure members.

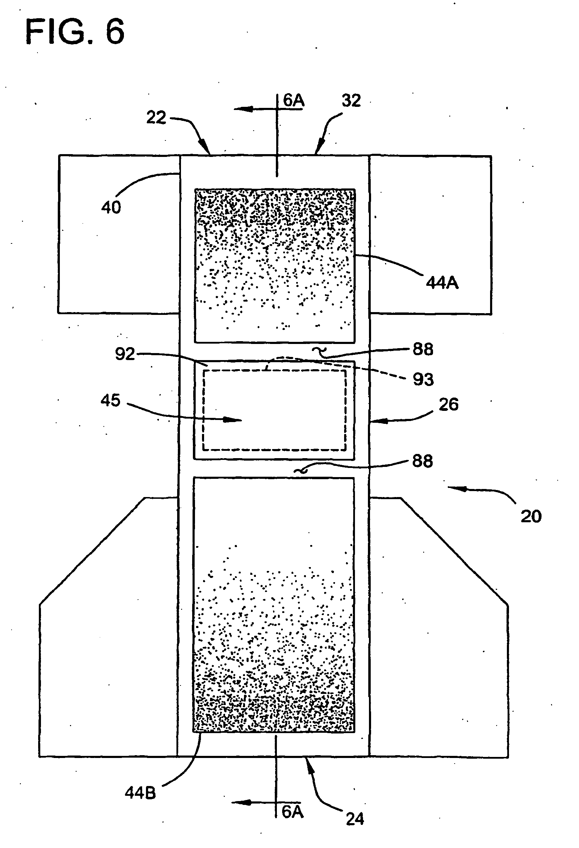

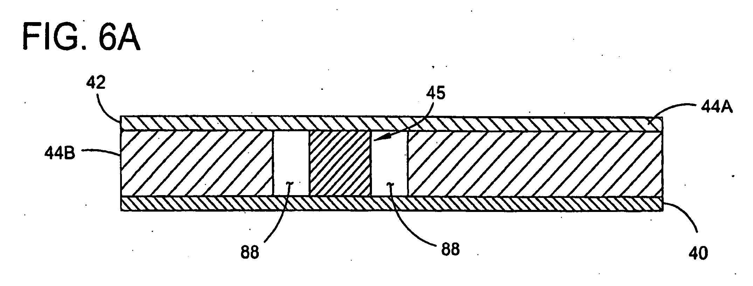

In another embodiment shown in Figs. 6 and 6a, the absorbent structure 44 is of two-piece construction similar having absorbent structure members 44A and 44B similar to those of Fig. 5. In this embodiment, the wetness indicator 45 is also disposed longitudinally between the absorbent structure members 44A, 44B in edge-facing-edge relationship, but the absorbent structure members are spaced longitudinally from the wetness indicator 45 to define respective gaps 88 between the longitudinal edges of the wetness indicator and the respective longitudinally inner edges of the absorbent structure members. As an example, each of the gaps 88 shown in Fig. 6 is suitably in the range of about 1 millimeter (mm) to about 30 mm, more suitably in the range of about 3 mm to about 10 mm, and even more suitably about 5 mm. While the gap 88 between the wetness indicator 45 and the front absorbent structure member 44A is shown in Figs. 6 and 6A as being substantially the same size as the gap between the wetness indicator and the back absorbent structure member 44B, it is understood that the sizes of the gaps may be different. It is also contemplated that only one of the absorbent structure members 44A, 44B may be longitudinally spaced from the wetness indicator 45 without departing from the scope of this invention.

The absorbent structure members 44A and 44B suitably each have a non-uniform absorbent capacity similar to the absorbent structure members of the embodiment of Fig. 5. That is, each of the absorbent structure members 44A and 44B has an absorbent capacity that increases from the edge nearest the wetness indicator longitudinally away from the position of the wetness indicator (e.g., toward the respective front and back regions of the pants 20). Alternatively, only one of the absorbent structure members 44A, 44B may have a non-uniform absorbent capacity, or neither of the absorbent structures may have a non-uniform absorbent capacity, without departing from the scope of this invention.

Upon the wearer's release of liquid body exudates into pants 20 incorporating the wetness indicator 45 and absorbent structure members 44A, 44B shown in Figs. 6 and 6a, the liquid permeates through the liner 42 and gravity tends to draw the liquid down toward the wetness indicator in the crotch region 26 of the training pants 20. The liquid tends to pool in the crotch region 26, and in particular in the gaps 88 between the wetness indicator 45 and the absorbent structure members 44A, 44B. As a result, a large portion of the liquid from the first insult is absorbed into the wetness indicator 45 rather than the absorbent structure members 44A, 44B. Also, isolating the wetness indicator 45 from the absorbent structure members 44A, 44B enhances tactile feedback to the wearer that urination has occurred. That is, as superabsorbent material in the absorbent structure 44 absorbs liquid, it expands, thereby expanding the absorbent structure. This expansion can cushion or mask the sensory effect of the wetness indicator 45. By separating the wetness indicator 45 from the absorbent structure members 44A, 44B, the risk of masking or cushioning by the absorbent structure members is reduced.

Separating the wetness indicator 45 from the absorbent structure members 44A, 44B also inhibits capillary attraction between the wetness indicator and the absorbent structure members. That is, it inhibits the formation of a capillary bridge between the wetness indicator 45 and the absorbent structure members 44A, 44B so that the absorbent structure members cannot draw urine back out of the wetness indicator. As a result, the stiffness of the wetness indicator 45 is more readily maintained once it is wet.

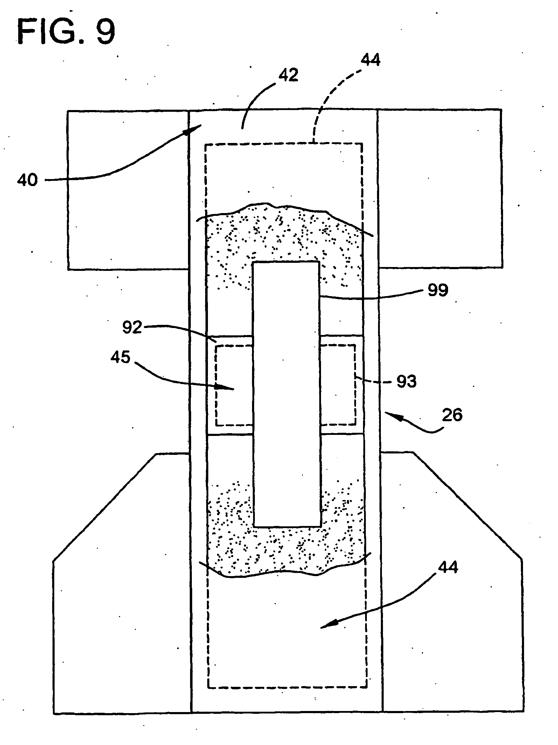

Figure 9 illustrates another embodiment of the training pants 20 in which a free-liquid holding member 99 is employed to decelerate and channel surges or gushes of liquid toward the wetness indicator 45. Desirably, the free-liquid holding member 99 can quickly take-in and temporarily hold the liquid prior to releasing the liquid to the wetness indicator 45. In the illustrated embodiment, the free-liquid holding member 99 is suitably disposed between the liner 42 and the wetness indicator 45 and extends longitudinally forward and rearward beyond the wetness indicator in overlaid relationship with the absorbent structure 44. Alternatively, the free-liquid holding member 99 may be located on top of the liner 44.

As with the wetness indicator 45, the longitudinal position of the free-liquid holding member 99 may be different depending on whether the training pants 20 are intended to be worn by girls or boys. For example, placement in a more forward position of the crotch region 26 may be appropriate for boys, while placement in a more central position between the legs may be appropriate for girls.

In one embodiment, the free-liquid holding member 99 suitably comprises a compressed cellulose sponge to provide a fluid holding zone which quickly collects the fluid and keeps it in the vicinity of the wetness indicator 45. As the wetness indicator 45 swells against the pressure provided by the constraining enclosure 92 and the high-absorbency-under load/high-gel-strength SAM of the absorbent body 93, it is relatively slow at absorbing liquid. The free-liquid holding member 99 keeps the wetness indicator 45 supplied with free fluid so that it can swell to the maximum allowed by the constraining enclosure.

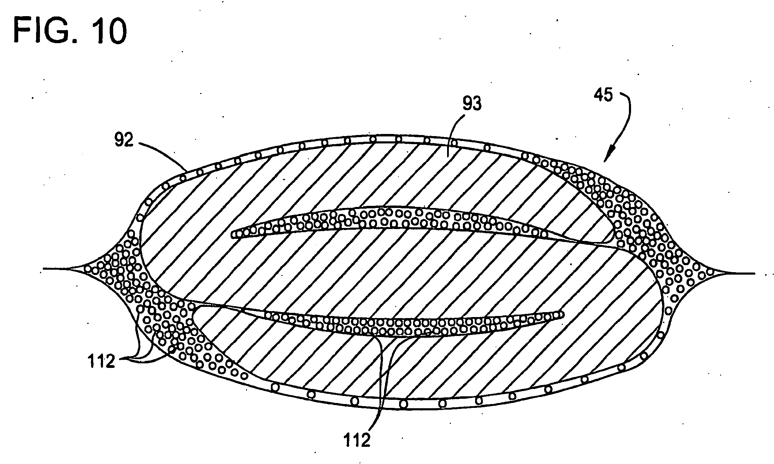

With reference now to Fig. 10, in addition to the wetness indicator 45 providing an increased stiffness to alert the wearer to urination, the pants 20 may further comprise a temperature change agent 112 that facilitates a tactile signal (e.g., a hot or cold sensation) against the wearer's skin to further alert the wearer that urination has occurred. While the temperature change agent 112 may be used with any of the embodiments described herein as having an absorbent structure 44 with an absorbent capacity gradient therein, it is understood that the temperature change agent 112 may be disposed in pants in which the absorbent structure is of generally uniform absorbent capacity or has an absorbent capacity other than that described previously herein. The temperature change can be caused by either an absorption or a release of heat by the temperature change agent 112 to change the temperature of the urine and hence surrounding components of the pants to a temperature noticeable to the wearer. For example, an absorption of heat by the temperature change agent 112 will provide a cool sensation against the wearer's skin while a release of heat by the temperature change agent will provide a warm sensation (e.g., warmer than the wearer's skin temperature) against the wearer's skin.

The temperature change agent 112 is suitably responsive to contact with an aqueous solution, such as urine, to either absorb or release heat. The mechanism by which this is accomplished may be the dissolution of the temperature change agent in the aqueous solution, the swelling of the agent in the aqueous solution and/or the reaction of the agent in the aqueous solution. In particular embodiments, the temperature change agent 112 is suitably in the form of particles which have a substantial energy difference between a dissolved state and a crystalline state, so that energy in the form of heat is absorbed or released to the environment upon contact with an aqueous solution such as urine. In other embodiments, the temperature change agent 112 releases or absorbs energy during swelling or reacting of the temperature change agent with an aqueous solution such as urine.

While a wide variety of temperature change agents 112 may result in a temperature change in response to contact with an aqueous solution, the selection of a particular temperature change agent and the determination of the amount to be used is based at least in part on the desired temperature change to be experienced by the wearer. For example, the temperature change agent 112 suitably provides a temperature change between the dry skin temperature prior to the urine insult and the skin temperature after the urine insult between about 5 and about 25 degrees Fahrenheit (°F) (2.8°C - 13.8°C). A further example of a suitable temperature change is a reduction in the urine temperature from an initial, body core temperature of about 98°F to 100°F (37°C - 38°C) to a cooled temperature of about 55°F to 75°F (13°C - 24°C), however, lowering the urine temperate to below 13°C may also be suitable in'the wetness indicator 45. To achieve this result, the temperature change agent 112, the amount used, and the location of the agent within the training pants is selected so that the potential total energy change is from about 6 to about 30 calories per square centimeter (cal/cm²), which may represent either a potential total energy release of from about 6 to about 30 cal/ cm² or a potential total energy absorption of from about 6 to about 30 cal/ cm². More suitably, the temperature change agent 112, the amount used, and the location of the substance within the pants is selected so that the potential total energy change is from about 12 to about 24 cal/ cm², and more particularly about 18 cal/ cm².

As referenced above, temperature change agents 112 suitable for use in the training pants 20 include those which dissolve in an aqueous solution. The solubility of such temperature change agents 112 is suitably in the range of about 0.1 to about 3 grams of water (H₂O) per gram of agent (g/g), and more particularly from about 0.1 to about 2 g/g.

In one particular embodiment, the temperature change agent 112 suitably comprises an endothermic material, which as used herein refers to any material that absorbs heat upon contact with an aqueous solution to provide a negative temperature change (e.g., a cooling of the aqueous solution). By way of illustration, suitable endothermic materials which absorb heat during dissolution upon contact with an aqueous solution include without limitation salt hydrates such as sodium acetate (H₂O), sodium carbonate (10H₂O), sodium sulfate (10H₂O), sodium thiosulfate (5H₂O), and sodium phosphate (10H₂O); anhydrous salts, such as ammonium nitrate, potassium nitrate, ammonium chloride, potassium chloride, sodium bromide, magnesium chloride, calcium chloride (6 H₂O), magnesium sulfate and sodium nitrate; organic compounds, such as urea, xylitol and other sugars; or the like.

In another embodiment, the temperature change agent 112 comprises a material that releases heat during dissolution, including without limitation aluminum chloride, aluminum sulfate, potassium aluminum sulfate; or the like.

The temperature change agent 112 may also, or may instead, comprise a material which absorbs or releases heat during swelling upon contact with aqueous solution. By way of illustration, one suitable material that releases heat during such swelling is.a lightly cross-linked partially neutralized polyacrylic acid. Alternatively, or additionally, the temperature change agent 112 may comprise a material which absorbs or releases heat upon reaction with an aqueous solution. Additional examples of suitable temperature change agents 112 are further described in co-assigned U.S. Patent No. 5,702,376 entitled TOILET TRAINING AID PROVIDING A TEMPERATURE AND DIMENSIONAL CHANGE SENSATION, issued December 30, 1997 to Glaug et al.

In the illustrated embodiment of Fig. 10, the temperature change agent 112 is in the form of endothermic material particles, such as urea particles, disposed within the liquid permeable enclosure 92 of the wetness indicator 45. More particularly, the particles are disposed between the enclosure 92 and the absorbent body 93 and/or between the folds (e.g., layers) of the absorbent body. The enclosure 92 and absorbent body 93 thereby contain or otherwise at least inhibit movement of the temperature change agent 112 particles within the wetness indicator when the wetness indicator 45 is dry. As an example, a suitable amount of urea particles enclosed within the enclosure of the wetness indicator 45 of Fig. 10 is in the range of about 5 to about 70 grams weight of urea particles. The selection of urea particles in this amount results in a drop in temperature on the surface of the wetness indicator 45 to about 65°F to 75°F (18°C - 24°C) after about 30 to 60 seconds after the urine void or insult with saline.

In use, as urine passes through the liquid permeable enclosure 92 of the wetness indicator 45 of Fig. 10, the urine comes into contact with and dissolves the temperature change agent 112 during or shortly after urination. The temperature change agent 112 absorbs heat from the urine upon dissolution and the cooled urine is then absorbed into the absorbent body 93. The wetness indicator 45 stiffens upon absorption of the cooled urine as described previously and applies pressure against the wearer's skin, such as against the wearer's inner thighs as described previously. The cooled wetness indicator 45 acts in the manner of a heat sink in thermally conductive contact with the wearer's skin (e.g., via the liner 42 and/or containment flaps of the pants 20) to thereby draw heat from the wearer and provide a cool sensation to the wearer's skin. Positioning the cooled wetness indicator in thermally conductive contact with the wearer for a significant duration of time allows the temperature change resulting from the temperature change agent 112 to be more easily noticed by the wearer.

The swelling and stiffening of the wetness indicator 45 thus facilitates a more direct thermally conductive contact between the cooled wetness indicator and the wearer's skin. Where air space exists between the article and the wearer's skin, as could happen in prior articles which rely on temperature change as an indicator of wetness, conductive heat transfer does not occur or is otherwise substantially reduced. The effects of convection and radiation are relatively insignificant. Use of the stiffening wetness indicator 45 to hold the cooled sensation against the wearer's skin reduces the ability of the wearer to escape thermally conductive contact with the heat sink (e.g., the cooled and stiffened wetness indicator 45) by shifting their body position.

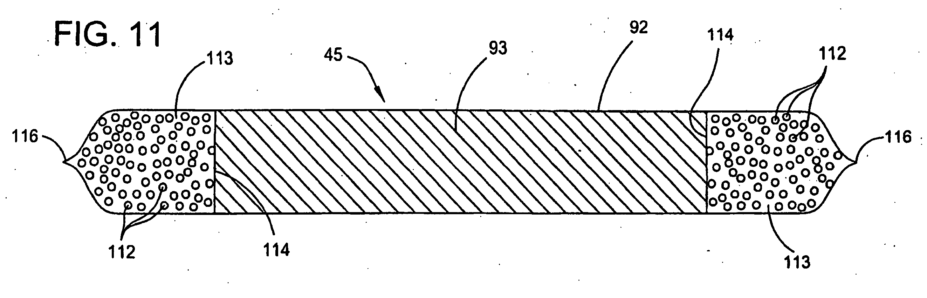

Figure 11 illustrates an alternative embodiment in which the temperature change agent 112 is disposed in separate compartments 113 formed at least in part by the enclosure 92 of the wetness indicator 45 generally at the lateral side edges thereof. Although not illustrated, wood pulp fluff, such as the previously mentioned CR1654, or other absorbing materials may be included in compartment 113 to help retain the cooled fluid within the compartment. In this embodiment, a panel 114 separates the compartment 113 from the absorbent body 93 of the wetness indicator 45 in which the absorbent body is located. The panel 114 is suitably constructed of a liquid impermeable material to inhibit cooled liquid formed in the compartments 113 against flowing into the absorbent body 93 of the wetness indicator 45 so that cooled urine tends to remain within the compartments. However, it is understood that the panel 114 may be liquid permeable so that some cooled liquid can flow into the absorbent body 93. In use, the separate compartments 113 located at the lateral side edges of the wetness indicator 45 concentrate the cooled liquid toward the laterally outer edges 116 of the wetness indicator 45 which are more directly in thermally conductive contact with the inner thighs of the wearer upon swelling and stiffening of the wetness indicator. Keeping the temperature change agent 112 separate from the absorbent body 93 within the wetness indicator 45 may also inhibit, or at least delay, salt from the temperature change agent 112 from contacting and negatively affecting the absorbent capacity of the superabsorbent material of the absorbent body.

Instead of the temperature change agent 112 being disposed within the wetness indicator 45 as is shown in the embodiments of Figs. 10 and 11, or in addition thereto, the temperature change agent may be disposed elsewhere within the pants, such as between the bodyside liner 42 and internal components such as the absorbent structure 44, wetness indicator 45, surge layer (if present) and/or any other layer or component disposed between the liner and the outer cover, and/or the temperature change agent 112 may be disposed within or otherwise on the liner. In such an embodiment, urine contacts the temperature change agent 112 during or shortly after urination, but before the urine flows through the liquid permeable enclosure 92 of the wetness indicator 45. The temperature change agent 112 thus dissolves and cools the urine prior to the urine passing through the liquid permeable enclosure of the wetness indicator 45 for absorption and swelling of the absorbent body within the wetness indicator.

Where the temperature change agent 112 is a salt, such as an endothermic salt, the superabsorbent material used in forming the absorbent body 93 of the wetness indicator 45 is suitably a bipolar superabsorbent absorbent material (bipolar SAM). Bipolar SAM refers to a class of superabsorbent materials comprising both anionic and cationic monomeric species within the absorbent composition. Bipolar SAM is not inactivated by electrolyte-containing liquids, such as urine, as much as conventional SAM. Rather, the absorbent capacity of bipolar SAM generally increases as the acid and amine groups are neutralized by ions from urine. Additionally, the bipolar SAM tends to increase the dissolution of endothermic salts used for the temperature change agent 112 by scavenging free ions from solution, therefore increasing the cooling effect since the ions in urine are depleted by this desalination effect of the bipolar SAM. In the context of this invention, an agent that scavenges or depletes ions from solution is any material that can measurably lower the level of free sodium ions in isotonic saline.

The bipolar SAM suitably contains at least one discrete microdomain of at least one acidic water-absorbing resin in contact with, or in close proximity to, at least one microdomain of at least one basic water-absorbing resin. The bipolar SAM particles can contain a plurality of microdomains of the acidic water-absorbing resin and/or the basic water-absorbing resin dispersed throughout the particle. As an example, suitable bipolar superabsorbent materials are disclosed in U.S. Patent No. 6,159,591 entitled MULTICOMPONENT SUPERABSORBENT GEL PARTICLES, issued December 12, 2000 to Beihoffer, et al.

In general, the monomeric species have been polymerized and crosslinked into water swellable but insoluble anionic and cationic polymers and then mixed together so that they interact when absorbing body liquids. The two polymeric types may interact as large domains such as individual particles of each species or as micro domains where the anionic and cationic polymeric domains comprise the same particle, existing on the tens to hundreds of micron scale. This class of absorbent polymer is typically not neutralized, as is the practice with conventional poly(acrylic acid) superabsorbents. Neutralization occurs when salt containing liquid contacts the bipolar absorbent composition with the anions from the salt associating with the cationic polymer component and the cations from the salt associating with the anionic polymer component.

The two polymer types in these samples are poly(acrylic acid) and poly(vinyl amine). Other examples of synthetic anionic polymers may include poly(methacrylic acid), hydrolyzed poly(acrylamides), maleic anhydride copolymers with vinyl ethers and alpha-olefins, poly(vinyl acetic acid), poly(vinyl sulfonic acid), poly(vinyl phosphonic acid), poly(vinyl ethers), poly(vinyl pyrrolidone), poly(vinylmorpholinone), poly(vinyl alcohol), and mixtures and copolymers thereof. Examples of natural based anionic polymers include carboxymethyl cellulose, carboxymethyl starch, alginates, and carrageenans. Other suitable examples of anionic polymers include synthetic polypeptides such as polyaspartic acid and polyglutamic acid. Alternative synthetic cationic polymers include poly(allylamines), poly(ethylene imine), poly(amino propanol vinyl ethers), poly(acrylamidopropyl trimethyl ammonium chloride), poly(diallyldimethyl ammonium chloride). Examples of natural based cationic polymers include partially deacetylated chitin, chitosan and chitosan salts. Also synthetic polypeptides such as polyasparagines, polylysines, polyglutamines, polyarginines can be examples of the cationic polymers.

In one embodiment where the absorbent body 93 of the wetness indicator 45 comprises non-bipolar SAM, the temperature change agent 112 is suitably free of sodium (which comprises mono-valent cations deleterious to the ability of non-bipolar SAM to absorb moisture and become stiff). More particularly, the temperature change agent 112 is suitably an endothermic salt having multi-valent cations, such as calcium, magnesium, and the like. Such a temperature change agent 112 may reduce the absorbent capacity of non-bipolar SAM as a result of increasing the cross-linking between the SAM molecular chains, but this cross-linking also tends to increase the stiffness of the SAM. Thus, the absorbent body 93 becomes stiffer upon absorbing urine, thereby enhancing the effectiveness of the wetness indicator 45.

Although the wetness indicator 45 is described herein primarily in the context of being used in children's training pants 20, the wetness indicator may also be used in conjunction with other articles, whether absorbent or non-absorbent and whether disposable or reusable, such as underwear or other reusable garments, diapers, medical garments, bandages, feminine care articles, adult incontinence articles and the like without departing from the scope of this invention.

When introducing elements of the present invention or the preferred embodiment(s) thereof, the articles "a", "an", "the", and "said" are intended to mean that there are one or more of the elements. The terms "comprising," "including", and "having" are intended to be inclusive and mean that there may be additional elements other than the listed elements.

## Claims

1. An article (20) for personal wear having a longitudinal axis (48) and a lateral axis (49), the article being capable of alerting a wearer to the wearer's release of liquid body exudates, said article comprising:
an absorbent structure (44) adapted to absorb said liquid body exudates, said absorbent structure having an absorbent capacity gradient along at least a portion thereof; and
a wetness indicator (45) adapted to receive said liquid body exudates, said wetness indicacor (45) being responsive to the liquid body exudates to apply a tactile sensation against the wearer's skin, said wetness indicator (45) being positioned relative to said portion of the absorbent structure (44) having an absorbent capacity gradient such that the absorbent capacity of the absorbent structure (44) increases as the absorbent structure extends generally from the position of the wetness indicator (45) in a direction generally longitudinally away from the position of the wetness indicator, wherein the absorbent capacity gradient of the absorbent structure (44) is defined at least in part by a density gradient of the absorbent structure (44) along said at least a portion thereof whereby the density of the absorbent structure increases as the absorbent structure extends generally from the position of the wetness indicator (45) in a direction generally longitudinally away from the position of the wetness indicator, or wherein the absorbent structure (44) comprises absorbent fibers and superabsorbent material, the absorbent capacity gradient of the absorbent structure (44) being defined at least in part by a superabsorbent material concentration gradient of the absorbent structure along said at least a portion thereof whereby the concentration of superabsorbent material within the absorbent structure increases as the absorbent structure extends generally from the position of the wetness indicator in a direction generally longitudinally away from the position of the wetness indicator.

2. The article of claim 1 wherein the wetness indicator is longitudinally positioned in the crotch region of the article.

3. An article (20) as set forth in claim 1 wherein the wetness indicator is arranged in generally edge-facing-edge relationship with the absorbent structure (44) whereby the absorbent capacity of the absorbent structure (44) increases as the absorbent structure extends from a longitudinal edge of the absorbent structure (44) proximate the wetness indicator (45) in a direction generally away from said edge.

4. An article (20) as set forth in claim 3 wherein the edge of the absorbent structure (44) proximate the wetness indicator (45) abuts the wetness indicator.

5. An article (20) as set forth in claim 3 wherein the absorbent structure (44) is in spaced relationship with the wetness indicator (45) to define a gap between the wetness indicator (45) and the longitudinal edge of the absorbent structure (44) proximate the wetness indicator.

6. An article (20) as set forth in claim 5 wherein said gap is in the range of about 5 millimeters to about 30 millimeters wide.

7. An article (20) as set forth in claim 3 wherein the absorbent structure (44) comprises first and second absorbent structure members (44A, 44B) spaced from each other, the wetness indicator (45) being disposed between said first and second absorbent structure members (44A, 44B) in generally edge-facing-edge relationship therewith, at least one of said first and second absorbent structure members having an absorbent capacity gradient wherein the absorbent capacity of the absorbent structure member increases from an edge of said at least one of the first and second absorbent structure members generally in a direction away from the wetness indicator (45).

8. An article (20) as set forth in claim 1 further comprising a liner (42) adapted for generally contiguous relationship with the wearer's skin during wearing of the article, and an outer cover (40), the wetness indicator (45) being disposed between the liner and the outer cover.

9. An article (20) as set forth in claim 1 wherein the article is configured for wear about a wearer's waist, said article having a front region (22), a back region (24) and a crotch region (26) interconnecting the front and back regions (22, 24) and extending generally longitudinally therebetween, the wetness indicator (45) being longitudinally positioned generally within the crotch region (26) of said article.

10. An article as set forth in claim 1 wherein a portion of the absorbent structure (44) at least one of at the position of the wetness indicator and generally adjacent thereto is substantially free of superabsorbent material.

11. An article as set forth in claim 1 further comprising a free-liquid holding member (99) arranged relative to the wetness indicator (45) and absorbent structure (44) for taking in liquid body exudates upon the release thereof by the wearer and channeling said liquid body exudates toward the wetness indicator.

12. An article as set forth in claim 11 wherein the free-liquid holding member (99) comprises a compressed sponge.

13. An article as set forth in claim 1 wherein the wetness indicator (45) comprises a liquid permeable enclosure (92) and an absorbent body (93) within said liquid permeable enclosure, said absorbent body (93) being capable of expansion upon the absorption of liquid body exudates thereby, said enclosure (92) limiting the expansion of the absorbent body (93) whereby the wetness indicator stiffens as liquid body exudates are absorbed by said absorbent body to apply a tactile sensation to the wearer's skin.

14. An article as set forth in claim 13 further comprising a temperature change agent (112) responsive to liquid body exudates received by the article to facilitate a temperature change sensation against the wearer's skin for alerting the wearer to the wearer's release of liquid body exudates.

15. An article as set forth in claim 14 wherein the absorbent body of the wetness indicator (45) at least in part comprises a bipolar superabsorbent material.

16. An article as set forth in claim 15 wherein the temperature change agent (112) is in the form of particles disposed at least one of on and within the liquid permeable enclosure (92) of the wetness-indicator structure.

17. An article as set forth in claim 15 wherein the temperature change agent (112) comprises an endothermic material.

18. An article as set forth in claim 17 wherein the endothermic material is a salt.

19. A disposable garment as set forth in claim 18 wherein the endothermic salt has multivalent cations.

20. An article as set forth in claim 14 wherein the absorbent body (93) of the wetness indicator at least in part contains an ion scavenging component that depletes ions from solution.

21. The article (20) of any preceding claim wherein the wetness indicator (45) is in overlaid relationship with the absorbent structure (44) or underlies the absorbent structure (44) or is enclosed within the absorbent structure.

## Patentansprüche

1. Artikel (20) zum persönlichen Gebrauch, der eine Längsachse (48) und eine Querachse (49) aufweist, wobei der Artikel in der Lage ist, einem Träger die Abgabe von flüssigen Körperausscheidungen des Trägers zu signalisieren, wobei der Artikel umfasst:
eine absorbtionsfähige Struktur (44), die dazu geeignet ist, die flüssigen Körperausscheidungen zu absorbieren, wobei die absorbtionsfähige Struktur einen Absorptionsfähigkeitsgradienten entlang mindestens eines Teils davon aufweist; und
einen Feuchtigkeitsanzeiger (45), der dazu geeignet ist, die flüssigen Körperausscheidungen aufzunehmen, wobei der Feuchtigkeitsanzeiger (45) auf die flüssigen Körperausscheidungen reagiert, so dass eine taktile Wahrnehmung auf der Haut des Trägers entsteht, wobei der Feuchtigkeitsanzeiger (45) in Bezug auf den Teil der absorbtionsfähigen Struktur (44) positioniert ist, die einen Absorptionsfähigkeitsgradienten derart aufweist, dass das Absorptionskapazität der absorbtionsfähigen Struktur (44) zunimmt, während sich die absorbtionsfähige Struktur im Allgemeinen von der Position des Feuchtigkeitsanzeigers (45) in eine Richtung erstreckt, die im Allgemeinen in Längsrichtung von der Position des Feuchtigkeitsanzeigers wegführt,
wobei der Absorptionskapazitätsgradient der absorbtionsfähigen Struktur (44) wenigstens zum Teil durch einen Dichtegradienten der absorbtionsfähigen Struktur (44) entlang des wenigstens einen Teils davon definiert wird, wobei die Dichte der absorbtionsfähigen Struktur zunimmt, während sich die absorbtionsfähige Struktur im Allgemeinen von der Position des Feuchtigkeitsanzeigers (45) in eine Richtung erstreckt, die im Allgemeinen in Längsrichtung von der Position des Feuchtigkeitsanzeigers wegführt, oder
wobei die absorbtionsfähige Struktur (44) absorbtionsfähige Fasern und superabsorbtionsfähiges Material umfasst, wobei der Absorptionskapazitätsgradient der absorbtionsfähigen Struktur (44) wenigstens zum Teil durch einen Konzentrationsgradient des superabsorbtionsfähigen Materials der absorbtionsfähigen Struktur entlang des wenigstens einen Teils davon definiert wird, wodurch die Konzentration von superabsorbtionsfähigem Material innerhalb der absorbtionsfähigen Struktur zunimmt, während sich die absorbtionsfähige Struktur im Allgemeinen von der Position des Feuchtigkeitsanzeigers in einer Richtung erstreckt, die im Allgemeinen in Längsrichtung von der Position des Feuchtigkeitsanzeigers wegführt.

2. Artikel nach Anspruch 1, wobei der Feuchtigkeitsanzeiger in Längsrichtung im Schrittbereich des Artikels positioniert ist.

3. Artikel (20) nach Anspruch 1, wobei der Feuchtigkeitsanzeiger in einer im Allgemeinen Kante-an-Kante-Beziehung zu der absorbtionsfähigen Struktur (44) angeordnet ist, wodurch die Absorptionskapazität der absorbtionsfähigen Struktur (44) zunimmt, während sich die absorbtionsfähige Struktur von einer Längskante der absorbtionsfähigen Struktur (44) nahe dem Feuchtigkeitsanzeiger (45) in einer im Allgemeinen von der Kante wegführenden Richtung erstreckt.

4. Artikel (20) nach Anspruch 3, wobei die Kante der absorbtionsfähigen Struktur (44) nahe dem Feuchtigkeitsanzeiger (45) an den Feuchtigkeitsanzeiger angrenzt.

5. Artikel (20) nach Anspruch 3, wobei die absorbtionsfähige Struktur (44) einen Abstand zum Feuchtigkeitsanzeiger (45) aufweist, um einen Zwischenraum zwischen dem Feuchtigkeitsanzeiger (45) und der Längskante der absorbtionsfähigen Struktur (44) nahe dem Feuchtigkeitsanzeiger zu definieren.

6. Artikel (20) nach Anspruch 5, wobei der Zwischenraum in einem Bereich von etwa 5 Millimetern bis etwa 30 Millimetern Breite liegt.

7. Artikel (20) nach Anspruch 3, wobei die absorbtionsfähige Struktur (44) erste und zweite absorptionsfähige Strukturelemente (44A, 44B) umfasst, die voneinander beabstandet sind, wobei der Feuchtigkeitsanzeiger (45) zwischen dem ersten und zweiten absorbtionsfähigen Strukturelement (44A, 44B) in einer im Allgemeinen Kante-an-Kante-Beziehung damit angeordnet ist, wobei wenigstens eines der ersten und zweiten absorbtionsfähigen Strukturelemente einen Absorptionskapazitätsgradienten aufweist, wobei die Absorptionskapazität des absorbtionsfähigen Strukturelements von einer Kante des wenigstens einen der ersten und zweiten absorbtionsfähigen Strukturelemente im Allgemeinen in einer von dem Feuchtigkeitsanzeiger (45) wegführenden Richtung zunimmt.

8. Artikel (20) nach Anspruch 1, der des Weiteren eine Auskleidung (42) umfasst, die für eine im Allgemeinen anliegende Beziehung zu der Haut des Trägers während des Tragens des Artikels geeignet ist, und eine äußere Deckschicht (40) umfasst, wobei der Feuchtigkeitsanzeiger (45) zwischen der Auskleidung und der äußeren Deckschicht angeordnet ist.

9. Artikel (20) nach Anspruch 1, wobei der Artikel für das Tragen um die Taille eines Trägers gestaltet ist, wobei der Artikel einen vorderen Bereich (22), einen hinteren Bereich (24) und einen Schrittbereich (26) aufweist, der den hinteren und den vorderen Bereich (22, 24) miteinander verbindet und sich im Allgemeinen in Längsrichtung dazwischen erstreckt, wobei der Feuchtigkeitsanzeiger (45) in Längsrichtung im Allgemeinen innerhalb des Schrittbereichs (26) des Artikels positioniert ist.

10. Artikel nach Anspruch 1, wobei ein Teil der absorbtionsfähigen Struktur (44), wenigstens einer davon, an der Position des Feuchtigkeitsanzeigers und im Allgemeinen daran angrenzend im Wesentlichen frei von superabsorbtionsfähigem Material ist.

11. Artikel nach Anspruch 1, der des Weiteren ein Auffangelement (99) für freie Flüssigkeit umfasst, das relativ zu dem Feuchtigkeitsanzeiger (45) und der absorbtionsfähigen Struktur (44) angeordnet ist, um flüssige Körperausscheidungen aufzunehmen, nachdem sie durch den Träger ausgeschieden wurden, und um die flüssigen Körperausscheidungen in Richtung des Feuchtigkeitsanzeigers zu leiten.

12. Artikel nach Anspruch 11, wobei das Auffangelement (99) für freie Flüssigkeit einen komprimierten Schwamm umfasst.

13. Artikel nach Anspruch 1, wobei der Feuchtigkeitsanzeiger (45) eine flüssigkeitsdurchlässige Einlage (92) und einen absorbtionsfähigen Körper (93) innerhalb der flüssigkeitsdurchlässigen Einlage umfasst, wobei der absorbtionsfähige Körper (93) in der Lage ist, sich nach der Absorption flüssiger Körperausscheidungen **dadurch** auszudehnen, und wobei die Einlage (92) die Ausdehnung des absorbtionsfähigen Körpers (93) begrenzt, wobei sich der Feuchtigkeitsanzeiger verfestigt, wenn flüssige Körperausscheidungen durch den absorbtionsfähigen Körper absorbiert werden, so dass eine taktile Wahrnehmung auf der Haut des Trägers entsteht.

14. Artikel nach Anspruch 13, der des Weiteren ein Temperaturänderungsmittel (112) umfasst, das auf flüssige Körperausscheidungen reagiert, die durch den Artikel empfangen werden, um ein Empfinden der Temperaturänderungswahrnehmung auf der Haut des Trägers zu ermöglichen, um den Träger auf das Ausscheiden von flüssigen Körperausscheidungen durch den Träger aufmerksam zu machen.

15. Artikel nach Anspruch 14, wobei der absorbtionsfähige Körper des Feuchtigkeitsanzeigers (45) wenigstens zum Teil ein bipolar superabsorbtionsfähiges Material umfasst.

16. Artikel nach Anspruch 15, wobei das Temperaturänderungsmittel (112) in Form von Teilchen vorhanden ist, von denen wenigstens eines auf und innerhalb der flüssigkeitsdurchlässigen Einlage (92) der Feuchtigkeitsanzeigestruktur angeordnet ist.

17. Artikel nach Anspruch 15, wobei das Temperaturänderungsmittel (112) ein endothermes Material umfasst.

18. Artikel nach Anspruch 17, wobei das endotherme Material ein Salz ist.

19. Einwegbekleidungsstück nach Anspruch 18, wobei das endotherme Salz mehrwertige Kationen aufweist.

20. Artikel nach Anspruch 14, wobei der absorbtionsfähige Körper (93) des Feuchtigkeitsanzeigers wenigstens zum Teil eine Ionenauffangkomponente enthält, die die Ionen der Lösung vermindert.

21. Artikel (20) eines der vorhergehenden Ansprüche, wobei sich der Feuchtigkeitsanzeiger (45) in überlagerndem Verhältnis zu der absorbtionsfähigen Struktur (44) befindet oder unter der absorbtionsfähigen Struktur (44) liegt oder in der absorbtionsfähigen Struktur eingeschlossen ist.

## Revendications

1. Article vestimentaire personnel (20) ayant un axe longitudinal (48) et un axe latéral (49), l'article étant capable d'avertir une personne qui le porte du fait qu'elle est en train de libérer des exsudats corporels liquides, ledit article comportant :
une structure absorbante (44) prévue pour absorber lesdits exsudats corporels liquides, ladite structure absorbante présentant un gradient de capacité absorbante le long d'au moins une partie de celle-ci ; et
un indicateur d'humidité (45) prévu pour recevoir lesdits exsudats corporels liquides, ledit indicateur d'humidité (45) réagissant aux exsudats corporels liquides pour appliquer une sensation tactile contre la peau de la personne portant l'article, ledit indicateur d'humidité (45) étant positionné par rapport à ladite partie de la structure absorbante (44) présentant un gradient de capacité absorbante de telle sorte que la capacité absorbante de la structure absorbante (44) augmente à mesure que la structure absorbante s'étend généralement à partir de la position de l'indicateur d'humidité (45) dans une direction s'éloignant généralement longitudinalement de la position de l'indicateur d'humidité, le gradient de capacité absorbante de la structure absorbante (44) étant défini au moins en partie par un gradient de densité de la structure absorbante (44) le long de ladite au moins une partie de celle-ci, la densité de la structure absorbante augmentant ainsi à mesure que la structure absorbante s'étend généralement à partir de la position de l'indicateur d'humidité (45) dans une direction s'éloignant généralement longitudinalement de la position de l'indicateur d'humidité, ou la structure absorbante (44) comportant des fibres absorbantes et un matériau superabsorbant, le gradient de capacité absorbante de la structure absorbante (44) étant défini au moins en partie par un gradient de concentration du matériau superabsorbant de la structure absorbante le long de ladite au moins une partie de celle-ci, la concentration de matériau superabsorbant à l'intérieur de la structure absorbante augmentant ainsi à mesure que la structure absorbante s'étend généralement à partir de la position de l'indicateur d'humidité dans une direction s'éloignant généralement longitudinalement de la position de l'indicateur d'humidité.

2. Article selon la revendication 1, l'indicateur d'humidité étant positionné longitudinalement dans la région d'entrejambe de l'article.

3. Article (20) selon la revendication 1, l'indicateur d'humidité étant disposé généralement bord à bord avec la structure absorbante (44), la capacité absorbante de la structure absorbante (44) augmentant ainsi à mesure que la structure absorbante s'étend à partir d'un bord longitudinal de la structure absorbante (44) proche de l'indicateur d'humidité (45) dans une direction s'éloignant généralement dudit bord.

4. Article (20) selon la revendication 3, le bord de la structure absorbante (44) proche de l'indicateur d'humidité (45) étant en contact avec l'indicateur d'humidité.

5. Article (20) selon la revendication 3, la structure absorbante (44) étant espacée de l'indicateur d'humidité (45) pour délimiter un intervalle entre l'indicateur d'humidité (45) et le bord longitudinal de la structure absorbante (44) proche de l'indicateur d'humidité.

6. Article (20) selon la revendication 5, ledit intervalle mesurant d'environ 5 millimètres à environ 30 millimètres de largeur.

7. Article (20) selon la revendication 3, la structure absorbante (44) comportant un premier élément de structure absorbante et un deuxième élément de structure absorbante (44A, 44B) espacés l'un de l'autre, l'indicateur d'humidité (45) étant disposé entre lesdits premier et deuxième éléments de structure absorbante (44A, 44B), généralement bord à bord par rapport à ceux-ci, au moins un desdits premier et deuxième éléments de structure absorbante présentant un gradient de capacité absorbante, la capacité absorbante de l'élément de structure absorbante augmentant à partir d'un bord dudit au moins un desdits premier et deuxième éléments de structure absorbante généralement dans une direction s'éloignant de l'indicateur d'humidité (45).

8. Article (20) selon la revendication 1, comportant en outre une doublure (42) prévue pour être en relation généralement contiguë avec la peau de la personne portant l'article pendant le port de l'article, et une couverture extérieure (40), l'indicateur d'humidité (45) étant disposé entre la doublure et la couverture extérieure.

9. Article (20) selon la revendication 1, l'article étant configuré pour être porté autour de la taille d'une personne portant l'article, ledit article comprenant une région avant (22), une région arrière (24) et une région d'entrejambe (26) reliant les régions avant et arrière (22, 24) et s'étendant de façon généralement longitudinale entre celles-ci, l'indicateur d'humidité (45) étant positionné longitudinalement généralement à l'intérieur de la région d'entrejambe (26) dudit article.

10. Article selon la revendication 1, une partie de la structure absorbante (44) au niveau et/ou généralement à proximité de la position de l'indicateur d'humidité étant sensiblement dépourvue de matériau superabsorbant.

11. Article selon la revendication 1, comportant en outre un élément de retenue de liquide libre (99) disposé par rapport à l'indicateur d'humidité (45) et à la structure absorbante (44) pour récupérer les exsudats corporels liquides suite à leur libération par la personne portant l'article et pour canaliser lesdits exsudats corporels liquides vers l'indicateur d'humidité.

12. Article selon la revendication 11, l'élément de retenue de liquide libre (99) comportant une éponge comprimée.

13. Article selon la revendication 1, l'indicateur d'humidité (45) comportant une enveloppe perméable aux liquides (92) et un corps absorbant (93) à l'intérieur de ladite enveloppe perméable aux liquides, ledit corps absorbant (93) étant susceptible de se dilater suite à l'absorption d'exsudats corporels liquides par celui-ci, ladite enveloppe (92) limitant la dilatation du corps absorbant (93), l'indicateur d'humidité se raidissant ainsi à mesure que des exsudats corporels liquides sont absorbés par ledit corps absorbant, pour appliquer une sensation tactile à la peau de la personne portant l'article.

14. Article selon la revendication 13, comportant en outre un agent de variation de température (112) réagissant aux exsudats corporels liquides reçus par l'article pour faciliter une sensation de variation de température contre la peau de la personne portant l'article afin d'avertir la personne portant l'article du fait qu'elle est en train de libérer des exsudats corporels liquides.

15. Article selon la revendication 14, le corps absorbant de l'indicateur d'humidité (45) consistant au moins en partie en un matériau superabsorbant bipolaire.

16. Article selon la revendication 15, l'agent de variation de température (112) se présentant sous la forme de particules disposées sur et/ou dans l'enveloppe perméable aux liquides (92) de la structure de l'indicateur d'humidité.

17. Article selon la revendication 15, l'agent de variation de température (112) comportant un matériau endothermique.

18. Article selon la revendication 17, le matériau endothermique étant un sel.

19. Vêtement jetable selon la revendication 18, le sel endothermique comprenant des cations multivalents.

20. Article selon la revendication 14, le corps absorbant (93) de l'indicateur d'humidité contenant au moins en partie un composant de captation d'ions qui appauvrit la solution en ions.

21. Article (20) selon l'une quelconque des revendications précédentes, l'indicateur d'humidité (45) étant superposé avec la structure absorbante (44), sous-jacent à la structure absorbante (44) ou enfermé à l'intérieur de la structure absorbante.
